# EUROPEAN PATENT APPLICATION

(11) **EP 4 606 894 A1**
(43) Date of publication of application: **27.08.2025**
(21) Application number: 23880250.8
(22) Date of filing: 19.10.2023
(51) Int. Cl.: C12N 9/22, C12N 9/78, C12N 15/62, C12N 15/10, C12N 15/113

(54) **FUSION PROTEIN CONTAINING CAS PROTEIN AND BACTERIAL TOXIN AND USE THEREOF**

(30) Priority: 19.10.2022 KR 20220134734
(71) Applicant: The Asan Foundation, Seoul 05505 (KR); UNIVERSITY OF ULSAN FOUNDATION FOR INDUSTRY COOPERATION, Nam-gu Ulsan 44610 (KR); Research & Business Foundation Sungkyunkwan University, Suwon-si, Gyeonggi-do 16419 (KR)
(72) Inventor: KIM, Yong Sub, Seoul 05505 (KR); KWON, Ji Yeon, Seoul 05505 (KR); KIM, Dae Sik, Suwon-si, Gyeonggi-do 16419 (KR)
(74) Representative: Bryn Aarflot AS
(86) International application number: PCT/KR2023/016230
(87) International publication number: WO 2024/085674

(57) **Abstract**

The present invention relates to a fusion protein including a Cas protein and a bacterial toxin and use thereof. The fusion protein, a polypeptide thereof, and a CRISPR-Cas system including the same according to an aspect may enable effective base editing. In addition, due to a small size of the polypeptide, the polypeptide facilitates easy delivery through vectors. In addition, when indels are accordingly induced, indel efficiency significantly increases, and for indel induction, the size of nucleotides where indels are formed also increases, whereby effective utilization can be made for gene knock-out.

## Description

### Technical Field

The present invention relates to a fusion protein containing a Cas protein and a bacterial toxin, and use thereof.

### Background Art

Genome editing is a technology for freely editing the genetic information of a living organism. Advances in the life sciences and development in genome sequencing technology have given people a broad understanding of diverse genetic information. For example, there has been already a clear understanding of genes for reproduction, disease and growth in animals and plants, genetic mutations that cause various human genetic diseases, production of biofuels, and the like, but technological advances beyond this understanding are essential to directly utilize these genes to improve living organisms and to reach the level of treating human diseases.

The entire editing technologies can dramatically expand the scope of applications by changing the genetic information of animals including humans, plants, and microorganisms. Gene scissors are molecular tools designed and manufactured to precisely cut the desired genetic information and play a key role in genome editing technologies. Like next-generation sequencing technology, which has advanced the field of genetic sequencing to the next level, the gene scissors are becoming a key technology that will expand the speed and scope of genetic information utilization and creates new industrial fields.

However, APOBEC or AID protein, which is a protein commonly used for cytosine base editing with Cas9, is relatively large, and thus is difficult to be used for the production of vectors for use as cell therapy agents. Accordingly, there is a demand for the production of a CRISPR-Cas system that is easy to produce as a vector and has excellent base editing efficiency.

### Disclosure of Invention

### Technical Problem

An aspect is to provide a fusion protein including a CRISPR-associated (Cas) protein and a bacterial toxin.

Another aspect is to provide a polynucleotide encoding the fusion protein.

Another aspect is to provide a vector including the polynucleotide.

Another aspect is to provide a CRISPR-Cas system including: the fusion protein or a polynucleotide encoding the fusion protein; and a guide polynucleotide.

Another aspect is to provide a method of editing a nucleic acid, the method including contacting a nucleic acid molecule with the CRISPR-Cas system.

### Solution to Problem

An aspect provides a fusion protein including a CRISPR-associated (Cas) protein and a bacterial toxin.

In the present specification, the term "CRISPR-associated (Cas) protein" may be a CRISPR-binding endonuclease. The Cas protein may cleave all or a part of a specific target polynucleotide sequence.

The Cas protein may be a class 2 Cas protein. The class 2 Cas protein may be included in a type II, type V, or type VI system.

The type II system may include cas1, cas2, and cas9 genes. The type II system may be further classified into three subtypes, namely subtypes II-A, II-B, and II-C. The subtype II-A may include an additional gene, csn2. An example of an organism that includes the subtype II-A system may include *Streptococcus thermophilus.* The subtype II-B lacks csn2, but may include cas4. An example of an organism that includes the subtype II-B system may include *Legionella pneumophila.* The subtype II-C is the most common type II system found in bacteria, and may have only three proteins, Cas1, Cas2, and Cas9. An example of an organism that includes the subtype II-C system may include *Neisseria lactamica.*

The type V system may include cas12 and cas 1 and cas2. The cas12 gene may encode a Cas12 protein, which has an RuvC-like nuclease domain homologous to each region of a Cas9 protein, but lacks an HNH nuclease domain present in the Cas9 protein.

The type VI system may include cas13 and cas1 and cas2.

In the type II system, each of an RuvC-like nuclease (RNase H fold domain) and an HNH (McrA-like) nuclease domain of Cas9 may cleave one of target nucleic acid strands. The Cas9 cleavage activity of the type II system may also require hybridization of crRNA and tracrRNA to form a duplex that promotes crRNA and target binding by Cas9.

In the type V system, a 5' overhang may be generated at the RuvC-like nuclease domain of Cas12 by cleavage of both strands of a target nucleic acid in a staggered configuration. This 5' overhang may facilitate DNA insertion via non-homologous end joining methods. The Cas12 cleavage activity of the type V system also does not require hybridization of crRNA and tracrRNA to form a duplex, and the crRNA of the type V system may use single crRNA with a stem- loop structure that forms an internal duplex. The type V system may induce breaks of a single strand or double strands at the location of a target sequence. Such a strand break may be a staggered cleavage with a 5' overhang.

The Cas protein may include Cas9 or Cas12.

The Cas12 protein may refer to a protein derived from various bacterial species. The Cas protein may be derived from the following genera: *Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium,* or *Acidaminococcus.* More specifically, the Cas12 protein may be derived from bacteria species selected from the group consisting of *F. tularensis 1, F. tularensis* subsp. *Novicida, P. albensis, Lachnospiraceae* bacterium MC2017 1, *Butyrivibrio proteoclasticus, Peregrinibacteria* bacterium GW2011_GWA2_33_10, *Parcubacteria* bacterium GW2011_GWC2_44_17, *Smitella* sp. SCADC, *Acidaminococcus* sp. BV3L6, *Lachnospiraceae* bacterium MA2020, *Candidatus Methanoplasma termitum, Eubacterium elligens, Moraxella bovoculi* 237, *Leptospira inadai, Lachnospiraceae* bacterium ND2006, *P. crevioricanis 3, P. diciens,* and *P.* macacae.

Additionally, the Cas12 protein may be any one selected from the group consisting of Cas12a, mgCas12a, Cas12b, Cas12c, Cas12d, Cas12e, Cas12f, Cas12g, Cas12h, Cas12i, and Cas12j. The Cas12 protein may include modifications of the Cas12 protein. When the Cas12 protein has nuclease activity, the Cas12 protein may be modified to have reduced nuclease activity, for example, nuclease inactivation of at least 70 %, at least 80 %, at least 90 %, at least 95 %, at least 97 %, or 100 %, compared to the wild-type enzyme.

The Cas9 may be Cas9 of S. *pneumoniae, S. pyogenes, S. thermophilus,* or *C. jejun,* and may include mutated Cas9 derived from these organisms. The enzyme may be a homologue or ortholog of the Cas9. In an embodiment, the CRISPR enzyme may be codon-optimized for expression in a eukaryotic cell. In an embodiment, the CRISPR enzyme may induce cleavage of one or both strands at the location of a target sequence. The Cas9 protein may include modifications of the Cas9 protein. When the Cas9 protein has nuclease activity, the Cas9 protein may be modified to have reduced nuclease activity, for example, nuclease inactivation of at least 70 %, at least 80 %, at least 90 %, at least 95 %, at least 97 %, or 100 %, compared to the wild-type enzyme. In an embodiment, the Cas9 may be Cas9 D10A.

In an embodiment the Cas protein may be the Cas9 protein or the Cas12 protein.

In an embodiment of the present invention, at least one nuclear localization signal (NLS) may be attached to a nucleic acid sequence encoding the Cas protein. In an embodiment, at least one NLS may be attached to the C-terminus or the N-terminus of the protein. The Cas protein, which includes at least one NLS, for example, about 1, 2, 3, 4, 5, 6, 7, 8, 9, 10, or more NLSs, or an ortholog or homologue thereof may be encoded. In a preferred embodiment regarding a Cas protein complex described in the present specification, a codon-optimized Cas protein may include an NLS attached to the C-terminus of the protein. In a specific embodiment, for example, other localization tags may be fused to the Cas protein to localize the Cas protein to specific sites within a cell, such as a cell organelle, e.g., a mitochondrion, a plastid, a chloroplast, a vesicle, a Golgi, a (nuclear or cellular) membrane, a ribosome, a nucleole, an ER, a cytoskeleton, a vacuole, a centrosome, a nucleosome, a granule, a centriole, etc. (but the sites are not limited thereto).

The Cas protein and the bacterial toxin may be fused via a linker. The linker may be located at the C-terminus, N-terminus, or both C-terminus and N-terminus of the Cas protein, and the bacterial toxin may be fused to the Cas protein via a linker. Suitable linker motifs and linker configurations include those described in the document [Chen et al., Fusion protein linkers: property, design and functionality. Adv Drug Deliv Rev. 2013; 65(10):1357-69], of which the entire contents are incorporated herein by reference.

In an embodiment, the bacterial toxin may be single-stranded DNA deaminase toxin A (SsdA).

The SsdA may be derived from strains of the genus *Pseudomonas SP.* The strains of the genus Pseudomonas may include *P. syringae, P. congelans, P. savastanoi, P. viridiflava, P. coronafaciens, P. fluorescens, Pseudomonas* sp. MPC6, *Pseudomonas* sp. GL-R-26, or *Pseudomonas* sp. GL-RE-26.

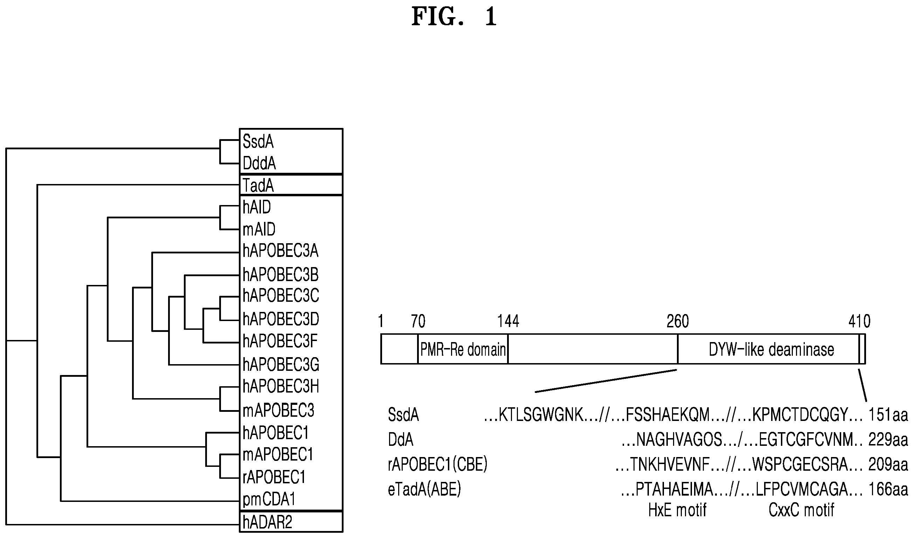

A PAAR domain may be found at the N-terminus of the SsdA, and a DYW deaminase domain may be found at the C-terminus of the SsdA. The SsdA is the same as a deaminase used for the existing base editing in that it has the common amino acid motifs, HxE and CxxC, but the SsdA differs from a deaminase used for the existing base editing in that it has an additional SGW motif. The SsdA is a deaminase that is structurally and evolutionarily different from other deaminases used in the existing base editing technology, and is classified as a DYW-like deaminase. The phylogenetic tree and differences in the constituent domains of the SsdA and other deaminases used in the existing base editing technology are shown in FIG. 1.

In an embodiment, the SsdA may include an amino acid sequence of SEQ ID NO: 1.

The SsdA may include a toxin domain. The toxin domain of the SsdA is a portion having deaminase activity, and may have a length of 100 to 200 amino acids, for example, a length of 120 to 180 amino acids, a length of 120 to 170 amino acids, a length of 130 to 160 amino acids, or a length of 140 to 160 amino acids. Specifically, the toxin domain of the SsdA may include an amino acid sequence of SEQ ID NO: 2. The base sequence of the toxin domain is shown in Table 1 below.

**[Table 1]**

| | Amino acid sequence | SEQ ID NO: |
|---|---|---|
| Toxin domain | KVSNIAESEAALGRASQARADLPQSKELKVKTVSSN DKKTLSGWGNKKPEGYERISAEQVKAKSEEIGHEVK SHPYDRDYKGQYFSSHAEKQMSIASPNHPLGVSKP MCTDCQGYFSQLAKYSKVEQTVADPKAIRIFKTDGS VETIMRSE | SEQ ID NO: 2 |

The amino acid sequence (toxin domain) of SEQ ID NO: 2 of the SsdA may include a catalytic active site. The catalytic active site may include an HxE motif, a CxxC motif, or an SGW motif. The SGW motif is a motif that only the SsdA additional has, unlike the existing deaminase enzymes such as APOBEC, AID, and the like. Specifically, the HxE motif may include amino acids at the 301st to 303rd positions, the HxE motif may include amino acids at the 347th to 349th positions, and the SGW motif may include amino acids at the 301st to 303rd positions, in SEQ ID NO: 1 (PAAR domain-containing protein).

In an embodiment, the SsdA may have a sequence identity of at least 80 %, 85 %, 90 %, 91 %, 92 %, 93 %, 94 %, 95 %, 96 %, 97 %, 98%, or 99 % with the amino acid sequence of SEQ ID NO: 1 (PAAR domain-containing protein).

In an embodiment, the fusion protein may be a DYW deaminase bound to a Cas protein.

In an embodiment, the SsdA may be inactivated SsdA.

The inactivated SsdA may be a result of an amino acid mutation occurring at the catalytic active site of activated SsdA. The inactivated SsdA may have low cytotoxicity compared to the SsdA.

In an embodiment, the inactivated SsdA may have an amino acid mutation at G302 and E349 positions in the amino acid sequence of SEQ ID NO: 1 (PAAR domain-containing protein). The amino acid mutation refers that a wild-type protein has been substituted with other amino acids, except for the amino acids at the G302 and E349 positions. The other amino acids may be any one selected from the group consisting of arginine (R), histidine (H), lysine (K), aspartic acid (D), glutamic acid (E), serine (S), threonine (T), asparagine (N), glutamine (Q), cysteine (C), selenocysteine (U), glycine (G), proline (P), alanine (A), valine (V), isoleucine (I), leucine (L), methionine (M), phenylalanine (F), tyrosine (Y), tryptophan (W), and all variants of these amino acids, excluding amino acids that a wild-type protein has at the mutation positions. Specifically, the inactivated SsdA may have G302D, E349A, or amino acid mutations corresponding thereto in the amino acid sequence of SEQ ID NO: 1. More specifically, the inactivated SsdA having a mutation of G302D in the amino acid sequence of the SEQ ID NO: 1 (PAAR domain-containing protein) may have SEQ ID NO: 17.

In an embodiment, the inactivated SsdA may have a sequence identity of at least 80 %, 85 %, 90 %, 91 %, 92 %, 93 %, 94 %, 95 %, 96 %, 97 %, 98 %, or 99 % with the amino acid sequence of SEQ ID NO: 17.

The SsdA may induce deamination of single-stranded DNA, and the SsdA may be a cytidine deaminase.

In the present specification, the term "cytidine deaminase" refers to an enzyme having the activity of removing the amino group (-NH₂) from cytosine, cytidine, or deoxycytidine. In the present specification, the cytidine deaminase is used as a concept including a cytosine deaminase. In the present specification, the cytidine deaminase may be used interchangeably with a cytosine deaminase.

The cytidine deaminase refers to any enzyme having the activity of converting cytosine, a base present in a nucleotide (e.g., cytosine present in DNA or RNA), to uracil (C-to-U conversion or C-to-U editing), wherein cytosine located on the strand where the PAM sequence of a target site (target nucleic acid sequence) is present is converted to uracil.

The bacterial toxin may be bound to the termini of the Cas protein. For example, the bacterial toxin may be bound to the C-terminus, the N-terminus, or both C-terminus and N-terminus of the Cas protein.

In an embodiment, the fusion protein may further include a DNA glycosylase inhibitor.

The DNA glycosylase inhibitor may be a thymine glycosylase inhibitor, an uracil glycosylase inhibitor, an oxoguanine glycosylase inhibitor, or an alkylguanine DNA glycosylase inhibitor.

The uracil DNA glycosylase inhibitor may be a uracil DNA glycosylase inhibitor derived from *Bacillus subtilis* bacteriophage PBS1 or a uracil DNA glycosylase inhibitor derived from *Bacillus subtilis* bacteriophage PBS2, but is not limited thereto.

Another aspect provides a polynucleotide encoding the fusion protein.

Another aspect provides a vector including the polynucleotide.

In the present specification, the term "vector" may refer to a nucleic acid molecule capable of transporting another nucleic acid to which it is linked. The vector may include: a single-stranded, double-stranded, or partially double-stranded nucleic acid molecule; a nucleic acid molecule including one or more free termini, a nucleic acid molecule without free termini (e.g., circular-type); a nucleic acid molecule including DNA, RNA, or both; and various other polynucleotides known in the art. One type of vector may be a "plasmid", which may refer to a circular double-stranded DNA loop, into which additional DNA fragments can be inserted by, for example, standard molecular cloning techniques. Another type of vector may be a viral vector, in which viral-derived DNA or RNA sequences may be present in the vector to be packaged into a virus (e.g., a retrovirus, a replication-defective retrovirus, an adenovirus, a replication-defective adenovirus, and an adeno-associated virus). A recombinant expression vector may include a nucleic acid of the present invention in a form suitable for expression of the nucleic acid in a host cell, meaning that the recombinant expression vector includes one or more regulatory elements, wherein the one or more regulatory elements may be selected based on a host cell to be used for expression and may operably be linked to a nucleic acid sequence to be expressed. The expression "operably linked" with reference to the recombinant expression vector may refer that a nucleotide sequence of interest is linked to regulatory element(s) in a manner that allows expression of the nucleotide sequence (for example, in an in vitro transcription/translation system or in a host cell if the vector has been introduced into a host cell).

In the present specification, the term "regulatory element" may include a promoter, an enhancer, an internal ribosomal entry site (IRES), and other expression control elements (e.g., a transcription termination signal, such as a polyadenylation signal and a poly-U sequence). For a description of the regulatory elements, reference may be made to, for example, the document [Goeddel, GENE EXPRESSION TECHNOLOGY: METHODS IN ENZYMOLOGY 185, Academic Press, San Diego, Calif. (1990)]. The regulatory element may include those that direct constitutive expression of a nucleotide sequence in many types of host cells and those that direct expression of a base sequence only in a certain host cell (e.g., tissue-specific regulatory sequences). In an embodiment, the vector may include one or more pol III promoters (e.g., 1, 2, 3, 4, 5 or more pol III promoters), one or more pol II promoters (e.g., 1, 2, 3, 4, 5 or more pol II promoters), one or more pol I promoters (e.g., 1, 2, 3, 4, 5 or more pol I promoters), or a combination thereof. Examples of the pol III promoters may include U6 and H1 promoter, but are not limited thereto. Examples of the pol II promoters may include a retrovirus Rous sarcoma virus (RSV) LTR promoter (optionally with an RSV enhancer), a cytomegalovirus (CMV) promoter (optionally with a CMV enhancer), an SV40 promoter, a dihydrofolate reductase promoter, a β-actin promoter, a phosphoglycerol kinase (PGK) promoter, and an EF1α promoter. For example, the vector may include lentiviruses and adeno-associated viruses (AAV1, AAV2, AAV3, AAV4, AAV5, AAV6, AAV7, AAV8, or AAV9), and the types of vector may also be selected for targeting specific types of cells.

In addition, multiple nucleic acid molecules within the vector system may be located on the same or different vectors.

In an embodiment, the vector, e.g., a plasmid or a viral vector, may be delivered to the tissue of interest, for example, by intramuscular injection, while in other cases delivery may be achieved via intravenous, transdermal, intranasal, oral, mucosal, or other delivery methods. Such delivery may be achieved via either a single dose or multiple doses. Those skilled in the art will appreciate that the actual dosage provided in the present specification may vary greatly depending on a variety of factors, such as vector selection, target cells, organisms, or tissues, or general conditions of a subject to be treated, the extent of desired transformation/modification, the route of administration, the method of administration, the form of desired transformation/modification. The dosage may further include, for example, a carrier (e.g., water, saline, ethanol, glycerol, lactose, sucrose, calcium phosphate, gelatin, dextran, agar, pectin, peanut oil, sesame oil, etc.), a diluent, a pharmaceutically acceptable carrier (e.g., phosphate buffered saline), a pharmaceutically acceptable excipient, and/or other compounds known in the art. The dosage may additionally include one or more pharmaceutically acceptable salts, for example, inorganic acid salts, such as hydrochlorides, hydrobromides, phosphates, sulfates, etc., and organic acid salts, such as acetates, propionates, malonates, benzoates, etc. Additionally, auxiliary ingredients, such as wetting or emulsifying agents, pH buffering agents, gels or gelling agents, flavoring agents, colorants, microspheres, polymers, suspending agents, etc. may also be provided in the present specification. Additionally, one or more other conventional pharmaceutical ingredients may also be present, such as preservatives, conditioning agents, suspending agents, surfactants, antioxidants, fillers, chelating agents, coating agents, chemical stabilizers, etc. Suitable exemplary ingredients may include microcrystalline cellulose, sodium carboxymethylcellulose, polysorbate 80, phenylethyl alcohol, chlorobutanol, potassium sorbate, sorbic acid, sulfur dioxide, propyl gallate, paraben, ethyl vanillin, glycerin, phenol, parachlorophenol, gelatin, albumin, and a combination thereof. For example, delivery for the treatment of a disease may be achieved via AAV. A therapeutically effective dosage for in vivo delivery of AAV to a human may be a saline solution in a range of about 20 ml to about 50 ml, containing about 1Y1010 to about 1Y10100 AAV per ml of solution. Such a dosage may be adjusted to balance the therapeutic benefit against any adverse effects.

Another aspect provides a CRISPR-Cas system including: a fusion protein including a Cas protein and a bacterial toxin or a polynucleotide encoding the fusion protein; and a guide polynucleotide.

The fusion protein and the polynucleotide encoding the same are as described above.

The guide polynucleotide may include a targeting sequence and/or an activating sequence.

As used in the present specification, the term "targeting sequence" may refer to a polynucleotide including DNA or a mixture of DNA and RNA, which is complementary to a sequence within a target nucleic acid. In a certain embodiment, the targeting sequence may also include other nucleic acids, or a nucleic acid analog, or a combination thereof. In a certain embodiment, the targeting sequence may consist solely of DNA, because the sequence configuration is less likely to be degraded inside the host cell. In an embodiment, the sequence configuration may increase target sequence recognition specificity and/or reduce the occurrence of off-target binding/hybridization. The targeting sequence may include a guide sequence or a spacer sequence. The length of a domain of the targeting sequence may be at least 12, 13, 14, 15, 16, 17, 18, 19, 20, 21, 22, 23, 24, 25, 26, 27, 28, 29, or 30 nucleotides in length.

In the present specification, the term "activating sequence" may refer to a portion of a polynucleotide including RNA, DNA, or a mixture of DNA and RNA, that is capable of interacting with, associating with, or binding to a Cas protein. In an embodiment, an activating region may also include other nucleic acids, or a nucleic acid analog, or a combination thereof. In an embodiment, the activating sequence may be adjacent to or linked to the target sequence. In an embodiment, the activating region may be downstream of a targeting region. In an embodiment, the activating region may be upstream of a targeting region. The activating sequence may include a direct repeat sequence, CRISPR RNA (crRNA), and/or trans-activating RNA (tracrRNA).

In an embodiment, the guide polynucleotide, guide RNA, mature crRNA, and immature crRNA may include or consist of a direct repeat sequence and a guide sequence or a spacer sequence. In an embodiment, the guide RNA or mature crRNA may include or consist of the direct repeat sequence linked to the guide sequence or the spacer sequence. In an embodiment, the direct repeat sequence may be located upstream (i.e., 5') from the guide sequence or the spacer sequence.

In an embodiment, the guide polynucleotide may include crRNA and tracrRNA.

In an embodiment, the guide polynucleotide may be dual guide RNA or single-chain guide RNA (sgRNA).

The system may form deletion, insertion, substitution, or insertion and deletion (indel) of at least one nucleotide of base sequences of a target nucleic acid molecule.

The nucleic acid may be RNA or DNA.

In an embodiment, the system may enable forming a deletion of a nucleotide of 1 bp to 60 bp, for example, 1 bp to 55 bp, 1 bp to 50 bp, 1 bp to 45 bp, 1 bp to 40 bp, 1 bp to 35 bp, 1 bp to 30 bp, 1 bp to 25 bp, 1 bp to 20 bp, 1 bp to 15 bp, 1 bp to 10 bp, 1 bp to 5 bp, 5 bp to 60 bp, 5 bp to 55 bp, 5 bp to 50 bp, 5 bp to 45 bp, 5 bp to 40 bp, 5 bp to 35 bp, 5 bp to 30 bp, 5 bp to 25 bp, 5 bp to 20 bp, 5 bp to 15 bp, 5 bp to 10 bp, 10 bp to 60 bp, 10 bp to 55 bp, 10 bp to 50 bp, 10 bp to 45 bp, 10 bp to 40 bp, 10 bp to 35 bp, 10 bp to 30 bp, 10 bp to 25 bp, 10 bp to 20 bp, 10 bp to 15 bp, 15 bp to 60 bp, 15 bp to 55 bp, 15 bp to 50 bp, 15 bp to 45 bp, 15 bp to 40 bp, 15 bp to 35 bp, 15 bp to 30 bp, 15 bp to 25 bp, 15 bp to 20 bp, 20 bp to 60 bp, 20 bp to 55 bp, 20 bp to 50 bp, 20 bp to 45 bp, 20 bp to 40 bp, 20 bp to 35 bp, 20 bp to 30 bp, 20 bp to 25 bp, 25 bp to 60 bp, 25 bp to 55 bp, 25 bp to 50 bp, 25 bp to 45 bp, 25 bp to 40 bp, 25 bp to 35 bp, 25 bp to 30 bp, 30 bp to 60 bp, 30 bp to 55 bp, 30 bp to 50 bp, 30 bp to 45 bp, 30 bp to 40 bp, 30 bp to 35 bp, 35 bp to 60 bp, 35 bp to 55 bp, 35 bp to 50 bp, 35 bp to 45 bp, 35 bp to 40 bp, 40 bp to 60 bp, 40 bp to 55 bp, 40 bp to 50 bp, 40 bp to 45 bp, 45 bp to 60 bp, 45 bp to 55 bp, 45 bp to 50 bp, 50 bp to 60 bp, 50 bp to 55 bp, or 55 bp to 60 bp, in a nucleotide sequence of a target nucleic acid molecule.

In an embodiment, the system may enable forming an insertion of a nucleotide of 1 bp to 60 bp, for example, 1 bp to 55 bp, 1 bp to 50 bp, 1 bp to 45 bp, 1 bp to 40 bp, 1 bp to 35 bp, 1 bp to 30 bp, 1 bp to 25 bp, 1 bp to 20 bp, 1 bp to 15 bp, 1 bp to 10 bp, 1 bp to 5 bp, 5 bp to 60 bp, 5 bp to 55 bp, 5 bp to 50 bp, 5 bp to 45 bp, 5 bp to 40 bp, 5 bp to 35 bp, 5 bp to 30 bp, 5 bp to 25 bp, 5 bp to 20 bp, 5 bp to 15 bp, 5 bp to 10 bp, 10 bp to 60 bp, 10 bp to 55 bp, 10 bp to 50 bp, 10 bp to 45 bp, 10 bp to 40 bp, 10 bp to 35 bp, 10 bp to 30 bp, 10 bp to 25 bp, 10 bp to 20 bp, 10 bp to 15 bp, 15 bp to 60 bp, 15 bp to 55 bp, 15 bp to 50 bp, 15 bp to 45 bp, 15 bp to 40 bp, 15 bp to 35 bp, 15 bp to 30 bp, 15 bp to 25 bp, 15 bp to 20 bp, 20 bp to 60 bp, 20 bp to 55 bp, 20 bp to 50 bp, 20 bp to 45 bp, 20 bp to 40 bp, 20 bp to 35 bp, 20 bp to 30 bp, 20 bp to 25 bp, 25 bp to 60 bp, 25 bp to 55 bp, 25 bp to 50 bp, 25 bp to 45 bp, 25 bp to 40 bp, 25 bp to 35 bp, 25 bp to 30 bp, 30 bp to 60 bp, 30 bp to 55 bp, 30 bp to 50 bp, 30 bp to 45 bp, 30 bp to 40 bp, 30 bp to 35 bp, 35 bp to 60 bp, 35 bp to 55 bp, 35 bp to 50 bp, 35 bp to 45 bp, 35 bp to 40 bp, 40 bp to 60 bp, 40 bp to 55 bp, 40 bp to 50 bp, 40 bp to 45 bp, 45 bp to 60 bp, 45 bp to 55 bp, 45 bp to 50 bp, 50 bp to 60 bp, 50 bp to 55 bp, or 55 bp to 60 bp, in a nucleotide sequence of a target nucleic acid molecule.

In an embodiment, the system may enable forming an indel of a nucleotide of 1 bp to 60 bp, for example, 1 bp to 55 bp, 1 bp to 50 bp, 1 bp to 45 bp, 1 bp to 40 bp, 1 bp to 35 bp, 1 bp to 30 bp, 1 bp to 25 bp, 1 bp to 20 bp, 1 bp to 15 bp, 1 bp to 10 bp, 1 bp to 5 bp, 5 bp to 60 bp, 5 bp to 55 bp, 5 bp to 50 bp, 5 bp to 45 bp, 5 bp to 40 bp, 5 bp to 35 bp, 5 bp to 30 bp, 5 bp to 25 bp, 5 bp to 20 bp, 5 bp to 15 bp, 5 bp to 10 bp, 10 bp to 60 bp, 10 bp to 55 bp, 10 bp to 50 bp, 10 bp to 45 bp, 10 bp to 40 bp, 10 bp to 35 bp, 10 bp to 30 bp, 10 bp to 25 bp, 10 bp to 20 bp, 10 bp to 15 bp, 15 bp to 60 bp, 15 bp to 55 bp, 15 bp to 50 bp, 15 bp to 45 bp, 15 bp to 40 bp, 15 bp to 35 bp, 15 bp to 30 bp, 15 bp to 25 bp, 15 bp to 20 bp, 20 bp to 60 bp, 20 bp to 55 bp, 20 bp to 50 bp, 20 bp to 45 bp, 20 bp to 40 bp, 20 bp to 35 bp, 20 bp to 30 bp, 20 bp to 25 bp, 25 bp to 60 bp, 25 bp to 55 bp, 25 bp to 50 bp, 25 bp to 45 bp, 25 bp to 40 bp, 25 bp to 35 bp, 25 bp to 30 bp, 30 bp to 60 bp, 30 bp to 55 bp, 30 bp to 50 bp, 30 bp to 45 bp, 30 bp to 40 bp, 30 bp to 35 bp, 35 bp to 60 bp, 35 bp to 55 bp, 35 bp to 50 bp, 35 bp to 45 bp, 35 bp to 40 bp, 40 bp to 60 bp, 40 bp to 55 bp, 40 bp to 50 bp, 40 bp to 45 bp, 45 bp to 60 bp, 45 bp to 55 bp, 45 bp to 50 bp, 50 bp to 60 bp, 50 bp to 55 bp, or 55 bp to 60 bp, in a nucleotide sequence of a target nucleic acid molecule.

In an embodiment, regarding the system, an efficiency of forming an indel in a nucleotide sequence may be from 5 % to 50%, for example, 5 % to 45 %, 5 % to 40 %, 5 % to 35%, 5 % to 30%, 5 % to 25 %, 5 % to 20%, 5 % to 15 %, 5 % to 10 %, 10 % to 50 %, 10% to 45 %, 10% to 40 %, 10 % to 35 %, 10% to 30 %, 10% to 25 %, 10 % to 20 %, 10 % to 15 %, 15 % to 50 %, 15 % to 45 %, 15 % to 40 %, 15 % to 35 %, 15 % to 30 %, 15 % to 25 %, 15 % to 20 %, 20 % to 50 %, 20 % to 45 %, 20 % to 40 %, 20 % to 35 %, 20 % to 30 %, 20 % to 25 %, 25 % to 50 %, 25 % to 45 %, 25 % to 40 %, 25 % to 35 %, 25 % to 30 %, 30 % to 50 %, 30 % to 45 %, 30 % to 40 %, 30 % to 35 %, 35 % to 50 %, 35 % to 45 %, 35 % to 40 %, 40 % to 50 %, 40 % to 45 %, or 45 % to 50 %.

**In** an embodiment, regarding the system, an efficiency of forming a substitution in a nucleotide sequence may be from 1 % to 20 %, for example, 1 % to 18 %, 1 % to 16 %, 1 % to 14 %, 1 % to 12 %, 1 % to 10 %, 1 % to 8 %, 1 % to 6 %, 1 % to 4 %, 1 % to 2 %, 2 % to 20 %, 2 % to 18 %, 2 % to 16 %, 2 % to 14 %, 2 % to 12 %, 2 % to 10 %, 2 % to 8 %, 2 % to 6 %, 2 % to 4 %, 4 % to 20 %, 4 % to 18 %,4 % to 16 %, 4 % to 14 %, 4 % to 12 %, 4 % to 10 %, 4 % to 8 %, 4 % to 6 %, 6 % to 20 %, 6 % to 18 %, 6 % to 16 %, 6 % to 14 %, 6 % to 12 %, 6 % to 10 %, 6 % to 8 %, 8 % to 20 %, 8 % to 18 %, 8 % to 16 %, 8 % to 14 %, 8 % to 12 %, 8 % to 10%, 10% to 20 %, 10% to 18 %, 10% to 16 %, 10% to 14 %, 10 % to 12 %, 12 % to 20 %, 12 % to 18 %, 12 % to 16 %, 12 % to 14 %, 14 % to 20%, 14 % to 18 %, 14 % to 16 %, 16 % to 20 %, 16 % to 18 %, or 18 % to 20 %.

By the system, an editing window of at least 4 nucleotides among the nucleotide sequence of a target nucleic acid molecule may be formed. **In an** embodiment, the system may have an editing window of at least 50 nucleotides, for example, at least 49 nucleotides, at least 48 nucleotides, at least 47 nucleotides, at least 46 nucleotides, at least 45 nucleotides, at least 44 nucleotides, at least 43 nucleotides, at least 42 nucleotides, at least 41 nucleotides, at least 40 nucleotides, at least 39 nucleotides, at least 38 nucleotides, at least 37 nucleotides, at least 36 nucleotides, at least 35 nucleotides, at least 34 nucleotides, at least 33 nucleotides, at least 32 nucleotides, at least 31 nucleotides, at least 30 nucleotides, at least 29 nucleotides, at least 28 nucleotides, at least 27 nucleotides, at least 26 nucleotides, at least 25 nucleotides, at least 24 nucleotides, at least 23 nucleotides, at least It may have an editing window of 22 nucleotides, at least 21 nucleotides, at least 20 nucleotides, at least 19 nucleotides, at least 18 nucleotides, at least 17 nucleotides, at least 16 nucleotides, at least 15 nucleotides, at least 14 nucleotides, at least 13 nucleotides, at least 12 nucleotides, at least 11 nucleotides, at least 10 nucleotides, at least 9 nucleotides, at least 8 nucleotides, at least 7 nucleotides, at least 6 nucleotides, at least 5 nucleotides, or at least 4 nucleotides.

In an embodiment, the system may have an editing window of 1 bp to 20 bp, 1 bp to 19 bp, 1 bp to 18 bp, 1 bp to 17 bp, 1 bp to 16 bp, 1 bp to 15 bp, 1 bp to 14 bp, 1 bp to 13 bp, 1 bp to 12 bp, 1 bp to 11 bp, 1 bp to 10 bp, 1 bp to 9 bp, 1 bp to 8 bp, 2 bp to 20 bp, 2 bp to 19 bp, 2 bp to 18 bp, 2 bp to 17 bp, 2 bp to 16 bp, 2 bp to 15 bp, 2 bp to 14 bp, 2 bp to 13 bp, 2 bp to 12 bp, 2 bp to 11 bp, 2 bp to 10 bp, 2 bp to 9 bp, 2 bp to 8 bp, 3 bp to 20 bp, 3 bp to 19 bp, 3 bp to 18 bp, 3 bp to 17 bp, 3 bp to 16 bp, 3 bp to 15 bp, 3 bp to 14 bp, 3 bp to 13 bp, 3 bp to 12 bp, 3 bp to 11 bp, 3 bp to 10 bp, 3 bp to 9 bp, 3 bp to 8 bp, 4 bp to 20 bp, 4 bp to 19 bp, 4 bp to 18 bp, 4 bp to 17 bp, 4 bp to 16 bp, 4 bp to 15 bp, 4 bp to 14 bp, 4 bp to 13 bp, 4 bp to 12 bp, 4 bp to 11 bp, 4 bp to 10 bp, 4 bp to 9 bp, or 4 bp to 8 bp, beginning from the 5'-terminus of the base sequence of target gRNA.

In an embodiment, the nucleotide editing window may refer to base editing in a range of first-positioned cytosine (C₁) to 20th-positioned cytosine (C₂₀), C₂ to C₂₀, C₃ to C₂₀, C₄ to C₂₀, C₁ to C₁₉, C₂ to C₁₉, C₃ to C₁₉, C₄ to C₁₉, C₁ to C₁₈, C₂ to C₁₈, C₃ to C₁₈, C₄ to C₁₈, C₁ to C₁₇, C₂ to C₁₇, C₃ to C₁₇, C₄ to C₁₇, C₁ to C₁₆, C₂ to C16, C₃ to C₁₆, C₄ to C₁₆, C₁ to C₁₅, C₂ to C₁₅, C₃ to C₁₅, C₄ to C₁₅, C₁ to C₁₄, C₂ to C14, C3to C₁₄, C₄ to C₁₄, C₁ to C₁₃, C₁ to C₁₃, C3to C₁₃, C₄ to C₁₃, C₁ to C₁₂, C₂ to C₁₂, C3to C₁₂, C₄ to C₁₂, C₁ to C₁₁, C₂ to C₁₁, C₃ to C₁₁, C₄ to C₁₁, C₁ to C₁₀, C₂ to C₁₀, C3to C₁₀, C₄ to C₁₀, C₁ to C₉, C₂ to C₉, C₃ to C₉, C₄ to C₉, C₁ to C₈, C₂ to C₈, C₃ to C₈ or C₄ to C₈, beginning from the 5'-terminus of the base sequence of target gRNA.

Another aspect provides a method of editing a nucleic acid, the method including contacting a nucleic acid molecule with a CRISPR-Cas system.

The nucleic acid and the CRISPR-Cas system are as described above.

In an embodiment, the editing may be to form a deletion, insertion, substitution, or indel of at least one nucleotide among the nucleotide sequences of the nucleic acid molecule.

In an embodiment, regarding the method of modifying the nucleic acid, the modification may include forming a deletion of 1 bp to 60 bp in a nucleotide sequence of the nucleic acid molecule, for example, 1 bp to 55 bp, 1 bp to 50 bp, 1 bp to 45 bp, 1 bp to 40 bp, 1 bp to 35 bp, 1 bp to 30 bp, 1 bp to 25 bp, 1 bp to 20 bp, 1 bp to 15 bp, 1 bp to 10 bp, 1 bp to 5 bp, 5 bp to 60 bp, 5 bp to 55 bp, 5 bp to 50 bp, 5 bp to 45 bp, 5 bp to 40 bp, 5 bp to 35 bp, 5 bp to 30 bp, 5 bp to 25 bp, 5 bp to 20 bp, 5 bp to 15 bp, 5 bp to 10 bp, 10 bp to 60 bp, 10 bp to 55 bp, 10 bp to 50 bp, 10 bp to 45 bp, 10 bp to 40 bp, 10 bp to 35 bp, 10 bp to 30 bp, 10 bp to 25 bp, 10 bp to 20 bp, 10 bp to 15 bp, 15 bp to 60 bp, 15 bp to 55 bp, 15 bp to 50 bp, 15 bp to 45 bp, 15 bp to 40 bp, 15 bp to 35 bp, 15 bp to 30 bp, 15 bp to 25 bp, 15 bp to 20 bp, 20 bp to 60 bp, 20 bp to 55 bp, 20 bp to 50 bp, 20 bp to 45 bp, 20 bp to 40 bp, 20 bp to 35 bp, 20 bp to 30 bp, 20 bp to 25 bp, 25 bp to 60 bp, 25 bp to 55 bp, 25 bp to 50 bp, 25 bp to 45 bp, 25 bp to 40 bp, 25 bp to 35 bp, 25 bp to 30 bp, 30 bp to 60 bp, 30 bp to 55 bp, 30 bp to 50 bp, 30 bp to 45 bp, 30 bp to 40 bp, 30 bp to 35 bp, 35 bp to 60 bp, 35 bp to 55 bp, 35 bp to 50 bp, 35 bp to 45 bp, 35 bp to 40 bp, 40 bp to 60 bp, 40 bp to 55 bp, 40 bp to 50 bp, 40 bp to 45 bp, 45 bp to 60 bp, 45 bp to 55 bp, 45 bp to 50 bp, 50 bp to 60 bp, 50 bp to 55 bp, or 55 bp to 60 bp in a nucleotide sequence of the nucleic acid molecule.

In an embodiment, regarding the method of modifying the nucleic acid, the modification may include forming an insertion of 1 bp to 60 bp in a nucleotide sequence of the nucleic acid molecule, for example, 1 bp to 55 bp, 1 bp to 50 bp, 1 bp to 45 bp, 1 bp to 40 bp, 1 bp to 35 bp, 1 bp to 30 bp, 1 bp to 25 bp, 1 bp to 20 bp, 1 bp to 15 bp, 1 bp to 10 bp, 1 bp to 5 bp, 5 bp to 60 bp, 5 bp to 55 bp, 5 bp to 50 bp, 5 bp to 45 bp, 5 bp to 40 bp, 5 bp to 35 bp, 5 bp to 30 bp, 5 bp to 25 bp, 5 bp to 20 bp, 5 bp to 15 bp, 5 bp to 10 bp, 10 bp to 60 bp, 10 bp to 55 bp, 10 bp to 50 bp, 10 bp to 45 bp, 10 bp to 40 bp, 10 bp to 35 bp, 10 bp to 30 bp, 10 bp to 25 bp, 10 bp to 20 bp, 10 bp to 15 bp, 15 bp to 60 bp, 15 bp to 55 bp, 15 bp to 50 bp, 15 bp to 45 bp, 15 bp to 40 bp, 15 bp to 35 bp, 15 bp to 30 bp, 15 bp to 25 bp, 15 bp to 20 bp, 20 bp to 60 bp, 20 bp to 55 bp, 20 bp to 50 bp, 20 bp to 45 bp, 20 bp to 40 bp, 20 bp to 35 bp, 20 bp to 30 bp, 20 bp to 25 bp, 25 bp to 60 bp, 25 bp to 55 bp, 25 bp to 50 bp, 25 bp to 45 bp, 25 bp to 40 bp, 25 bp to 35 bp, 25 bp to 30 bp, 30 bp to 60 bp, 30 bp to 55 bp, 30 bp to 50 bp, 30 bp to 45 bp, 30 bp to 40 bp, 30 bp to 35 bp, 35 bp to 60 bp, 35 bp to 55 bp, 35 bp to 50 bp, 35 bp to 45 bp, 35 bp to 40 bp, 40 bp to 60 bp, 40 bp to 55 bp, 40 bp to 50 bp, 40 bp to 45 bp, 45 bp to 60 bp, 45 bp to 55 bp, 45 bp to 50 bp, 50 bp to 60 bp, 50 bp to 55 bp, or 55 bp to 60 bp in a nucleotide sequence of the nucleic acid molecule.

In an embodiment, regarding the method of modifying the nucleic acid, the modification may include forming an indel of 1 bp to 60 bp in a nucleotide sequence of the nucleic acid molecule, for example, 1 bp to 55 bp, 1 bp to 50 bp, 1 bp to 45 bp, 1 bp to 40 bp, 1 bp to 35 bp, 1 bp to 30 bp, 1 bp to 25 bp, 1 bp to 20 bp, 1 bp to 15 bp, 1 bp to 10 bp, 1 bp to 5 bp, 5 bp to 60 bp, 5 bp to 55 bp, 5 bp to 50 bp, 5 bp to 45 bp, 5 bp to 40 bp, 5 bp to 35 bp, 5 bp to 30 bp, 5 bp to 25 bp, 5 bp to 20 bp, 5 bp to 15 bp, 5 bp to 10 bp, 10 bp to 60 bp, 10 bp to 55 bp, 10 bp to 50 bp, 10 bp to 45 bp, 10 bp to 40 bp, 10 bp to 35 bp, 10 bp to 30 bp, 10 bp to 25 bp, 10 bp to 20 bp, 10 bp to 15 bp, 15 bp to 60 bp, 15 bp to 55 bp, 15 bp to 50 bp, 15 bp to 45 bp, 15 bp to 40 bp, 15 bp to 35 bp, 15 bp to 30 bp, 15 bp to 25 bp, 15 bp to 20 bp, 20 bp to 60 bp, 20 bp to 55 bp, 20 bp to 50 bp, 20 bp to 45 bp, 20 bp to 40 bp, 20 bp to 35 bp, 20 bp to 30 bp, 20 bp to 25 bp, 25 bp to 60 bp, 25 bp to 55 bp, 25 bp to 50 bp, 25 bp to 45 bp, 25 bp to 40 bp, 25 bp to 35 bp, 25 bp to 30 bp, 30 bp to 60 bp, 30 bp to 55 bp, 30 bp to 50 bp, 30 bp to 45 bp, 30 bp to 40 bp, 30 bp to 35 bp, 35 bp to 60 bp, 35 bp to 55 bp, 35 bp to 50 bp, 35 bp to 45 bp, 35 bp to 40 bp, 40 bp to 60 bp, 40 bp to 55 bp, 40 bp to 50 bp, 40 bp to 45 bp, 45 bp to 60 bp, 45 bp to 55 bp, 45 bp to 50 bp, 50 bp to 60 bp, 50 bp to 55 bp, or 55 bp to 60 bp in a nucleotide sequence of the nucleic acid molecule.

In an embodiment, regarding the method of modifying the nucleic acid, an efficiency of forming an indel in a nucleotide sequence may be from 5 % to 50%, for example, 5 % to 45 %, 5 % to 40 %, 5 % to 35 %, 5 % to 30 %, 5 % to 25 %, 5 % to 20 %, 5 % to 15 %, 5 % to 10 %, 10 % to 50 %, 10 % to 45 %, 10 % to 40 %, 10 % to 35 %, 10 % to 30 %, 10% to 25 %, 10 % to 20 %, 10 % to 15 %, 15 % to 50 %, 15 % to 45 %, 15 % to 40 %, 15 % to 35 %, 15 % to 30 %, 15 % to 25 %, 15 % to 20 %, 20 % to 50 %, 20 % to 45 %, 20 % to 40 %, 20 % to 35 %, 20 % to 30 %, 20 % to 25 %, 25 % to 50 %, 25 % to 45 %, 25 % to 40 %, 25 % to 35 %, 25 % to 30 %, 30 % to 50 %, 30 % to 45 %, 30 % to 40 %, 30 % to 35 %, 35 % to 50 %, 35 % to 45 %, 35 % to 40 %, 40 % to 50 %, 40 % to 45 %, or 45 % to 50 %.

In an embodiment, regarding the method of modifying the nucleic acid, an efficiency of forming a substitution in a nucleotide sequence may be from 1 % to 20 %, for example, 1 % to 18 %, 1 % to 16 %, 1 % to 14 %, 1 % to 12 %, 1 % to 10 %, 1 % to 8 %, 1 % to 6 %, 1 % to 4 %, 1 % to 2 %, 2 % to 20%, 2 % to 18 %, 2 % to 16 %, 2 % to 14 %, 2 % to 12 %, 2 % to 10%, 2 % to 8 %, 2 % to6 %, 2 % to 4 %, 4 % to 20 %, 4 % to 18 %, 4 % to 16 %, 4 % to 14 %, 4 % to 12 %, 4 % to 10 %, 4 % to 8 %, 4 % to 6 %, 6 % to 20 %, 6 % to 18 %, 6 % to 16 %, 6 % to 14 %, 6 % to 12 %, 6 % to 10 %, 6 % to 8 %, 8 % to 20 %, 8 % to 18 %, 8 % to 16 %, 8 % to 14 %, 8 % to 12 %, 8 % to 10 %, 10% to 20 %, 10 % to 18 %, 10 % to 16 %, 10 % to 14 %, 10% to 12 %, 12 % to 20 %, 12 % to 18 %, 12 % to 16 %, 12 % to 14 %, 14 % to 20 %, 14 % to 18 %, 14 % to 16 %, 16 % to 20 %, 16 % to 18 %, or 18 % to 20 %.

By the method of modifying the nucleic acid, an editing window of at least 4 nucleotides among the nucleotide sequence of a target nucleic acid molecule may be formed. **In** an embodiment, regarding the method of modifying the nucleic acid, an editing window of at least 50 nucleotides, for example, at least 49 nucleotides, at least 48 nucleotides, at least 47 nucleotides, at least 46 nucleotides, at least 45 nucleotides, at least 44 nucleotides, at least 43 nucleotides, at least 42 nucleotides, at least 41 nucleotides, at least 40 nucleotides, at least 39 nucleotides, at least 38 nucleotides, at least 37 nucleotides, at least 36 nucleotides, at least 35 nucleotides, at least 34 nucleotides, at least 33 nucleotides, at least 32 nucleotides, at least 31 nucleotides, at least 30 nucleotides, at least 29 nucleotides, at least 28 nucleotides, at least 27 nucleotides, at least 26 nucleotides, at least 25 nucleotides, at least 24 nucleotides, at least 23 nucleotides, at least It may have an editing window of 22 nucleotides, at least 21 nucleotides, at least 20 nucleotides, at least 19 nucleotides, at least 18 nucleotides, at least 17 nucleotides, at least 16 nucleotides, at least 15 nucleotides, at least 14 nucleotides, at least 13 nucleotides, at least 12 nucleotides, at least 11 nucleotides, at least 10 nucleotides, at least 9 nucleotides, at least 8 nucleotides, at least 7 nucleotides, at least 6 nucleotides or at least 5 nucleotides, may be formed.

In an embodiment, regarding the method of modifying the nucleic acid, an editing window of 1 bp to 20 bp, 1 bp to 19 bp, 1 bp to 18 bp, 1 bp to 17 bp, 1 bp to 16 bp, 1 bp to 15 bp, 1 bp to 14 bp, 1 bp to 13 bp, 1 bp to 12 bp, 1 bp to 11 bp, 1 bp to 10 bp, 1 bp to 9 bp, 1 bp to 8 bp, 2 bp to 20 bp, 2 bp to 19 bp, 2 bp to 18 bp, 2 bp to 17 bp, 2 bp to 16 bp, 2 bp to 15 bp, 2 bp to 14 bp, 2 bp to 13 bp, 2 bp to 12 bp, 2 bp to 11 bp, 2 bp to 10 bp, 2 bp to 9 bp, 2 bp to 8 bp, 3 bp to 20 bp, 3 bp to 19 bp, 3 bp to 18 bp, 3 bp to 17 bp, 3 bp to 16 bp, 3 bp to 15 bp, 3 bp to 14 bp, 3 bp to 13 bp, 3 bp to 12 bp, 3 bp to 11 bp, 3 bp to 10 bp, 3 bp to 9 bp, 3 bp to 8 bp, 4 bp to 20 bp, 4 bp to 19 bp, 4 bp to 18 bp, 4 bp to 17 bp, 4 bp to 16 bp, 4 bp to 15 bp, 4 bp to 14 bp, 4 bp to 13 bp, 4 bp to 12 bp, 4 bp to 11 bp, 4 bp to 10 bp, 4 bp to 9 bp, or 4 bp to 8 bp, beginning from the 5'-terminus of the base sequence of target gRNA, may be formed.

In an embodiment, an editing window used in the method of modifying the nucleic acid may refer to base editing in a range of first-positioned cytosine (C₁) to 20th-positioned cytosine (C₂₀), C₂ to C₂₀, C₃ to C₂₀, C₄ to C₂₀, C₁ to C₁₉, C₂ to C₁₉, C₃ to C₁₉, C₄ to C₁₉, C₁ to C₁₈, C₂ to C₁₈, C_{3 to} C₁₈, C₄ to C₁₈, C₁ to C₁₇, C₂ to C17, C₃ to C₁₇, C₄ to C₁₇, C₁ to C₁₆, C₂ to C₁₆, C_{3 to} C₁₆, C₄ to C₁₆, C₁ to C₁₅, C₂ to C₁₅, C₃ to C₁₅, C₄ to C₁₅, C₁ to C₁₄, C₂ to C₁₄, C₃ to C₁₄, C₄ to C₁₄, C₁ to C₁₃, C₂ to C₁₃, C₃ to C13, C₄ to C₁₃, C₁ to C₁₂, C₂ to C₁₂, C₃ to C₁₂, C₄ to C₁₂, C₁ to C₁₁, C₂ to C₁₁, C₃ to C₁₁, C4 to C₁₁, C₁ to C₁₀, C₂ to C₁₀, C₃ to C₁₀, C₄ to C₁₀, C₁ to C₉, C₂ to C₉, C₃ to C₉, C₄ to C₉, C₁ to C₈, C₂ to C₈, C₃ to C₈ or C₄ to C₈, beginning from the 5'-terminus of the base sequence of target gRNA.

### Advantageous Effects of Invention

According to the fusion protein of an aspect, the polypeptide thereof, and the CRISPR-Cas system including the fusion protein, effective base editing may be performed. In addition, the small size of the polypeptide facilitates binding to the Cas protein, and the polypeptide exhibits an effect of easy delivery through a vector when used as a cell therapy agent.

In addition, when indels are induced through the polypeptide, the indel efficiency and the size of the nucleotide in which the indels are formed also increase, and thus the polypeptide can be effectively utilized for gene knock-out.

### Brief Description of Drawings

FIG. 1 is a schematic diagram showing the structural/evolutionary differences between SsdA and other deaminases used in the existing base editing technologies.
FIG. 2 is a diagram describing a process of deaminating single-stranded DNA having the base sequence of SEQ ID NO: 4 by treatment with SsdA and cleaving the single-stranded DNA by treatment with UDG and NaOH, and shows an image showing the results of western blot to confirm deamination of the SsdA.
FIG. 3 is a graph showing base editing efficiency of a CRISPR-Cas system at a target site of target DNA, the CRISPR-Cas system including a Cas protein, SsdA, and a guide polynucleotide; FIG. 3A is a graph showing base editing efficiency of a CRISPR-Cas system, which includes a Cas protein, SsdA, and a guide polynucleotide, at a target site of RNF2 DNA, and FIG. 3B is a graph showing base editing efficiency of a CRISPR-Cas system, which includes a Cas protein, SsdA, and a guide polynucleotide, at a target site of HEK2 DNA.
FIG. 4 is a graph showing cytotoxicity of each of a Cas9(D10A)-SsdA fusion protein, a Cas9(D10A)-SsdA-UGI fusion protein, a dCas9-SsdA fusion protein, and a dCas9-SsdA-UGI fusion protein.
FIG. 5 is a graph showing base editing efficiency and indel formation efficiency of a CRISPR-Cas system including a Cas9-SsdA fusion protein:

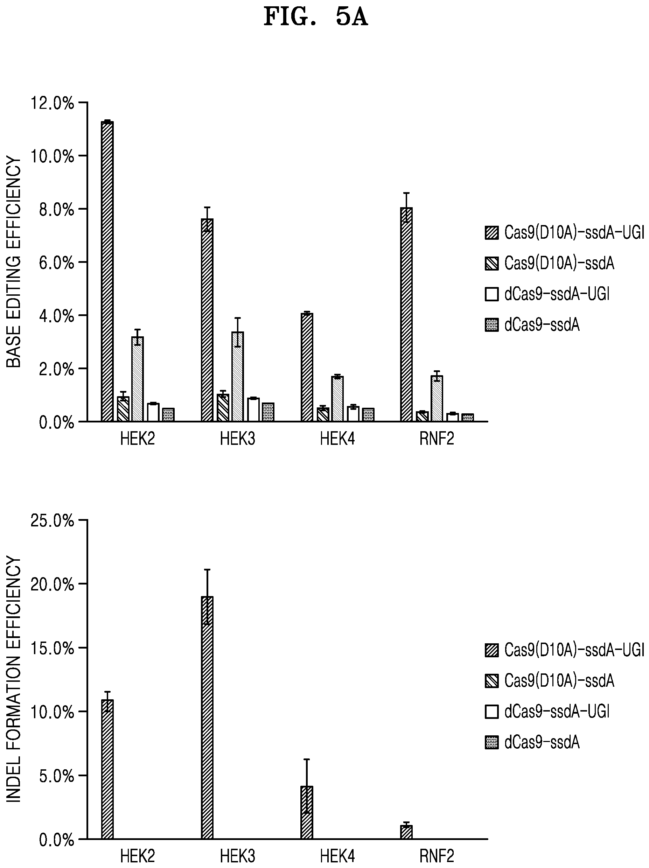

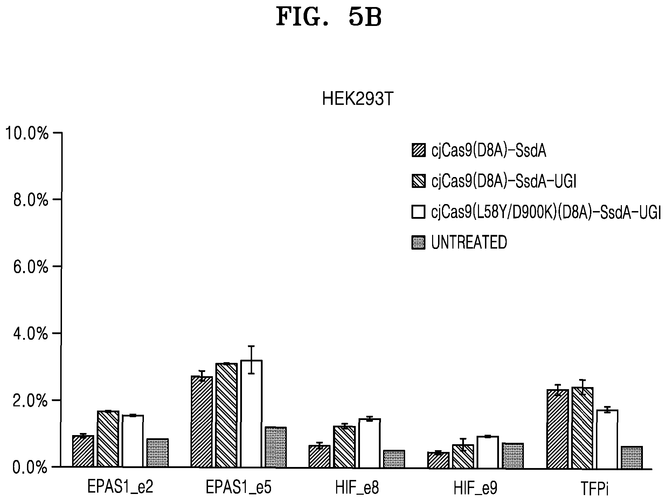

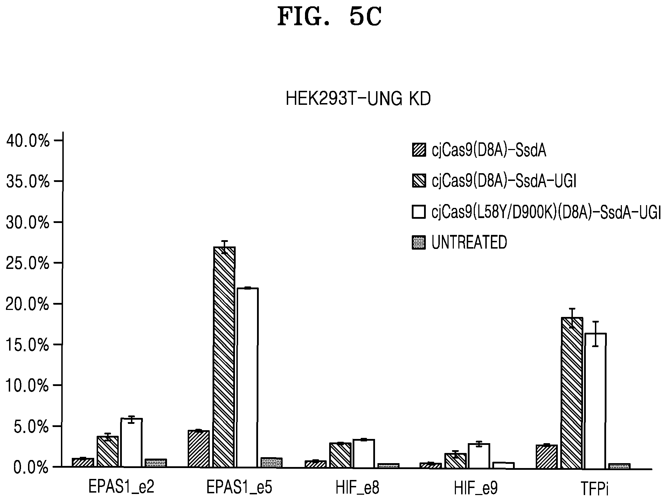

   FIG. 5A is a graph showing base editing efficiency and indel formation efficiency of each CRISPR-Cas system including a Cas9(D10A)-SsdA fusion protein, a Cas9(D10A)-SsdA-UGI fusion protein, a dCas9-SsdA fusion protein, or a dCas9-SsdA-UGI fusion protein, at HEK2, HEK3, HEK4, and RNF2; FIG. 5B is a graph showing base editing efficiency and indel formation efficiency of each CRISPR-Cas system including a cjCas9(D8A)-SsdA fusion protein, a cjCas9(D8A)-SsdA-UGI fusion protein, or a cjCas9(L58Y/D900K)(D8A)-SsdA-UGI fusion protein, at EPAS1_e2, EPAS1_e5, HIF_e8, HIF_e9, and TFPi; and FIG. 5C is a graph showing base editing efficiency and indel formation efficiency of each CRISPR-Cas system including a cjCas9(D8A)-SsdA fusion protein, a cjCas9(D8A)-SsdA-UGI fusion protein, or ca jCas9(L58Y/D900K)(D8A)-SsdA-UGI fusion protein, at EPAS1_e2, EPAS1_e5, HIF_e8, HIF_e9, and TFPi in uracil-DNA glycosylase (UDG) expression knockdown cell line.
   FIG. 6 is a schematic diagram of a plasmid for producing a fusion protein in which SsdA and uracil glycosylase inhibitor (UGI) are bound to the C-terminus, the N-terminus, and both the N-terminus and the C-terminus of a Cas protein.
   FIG. 7 is a graph confirming base editing efficiency according to binding location of SsdA and a Cas protein.

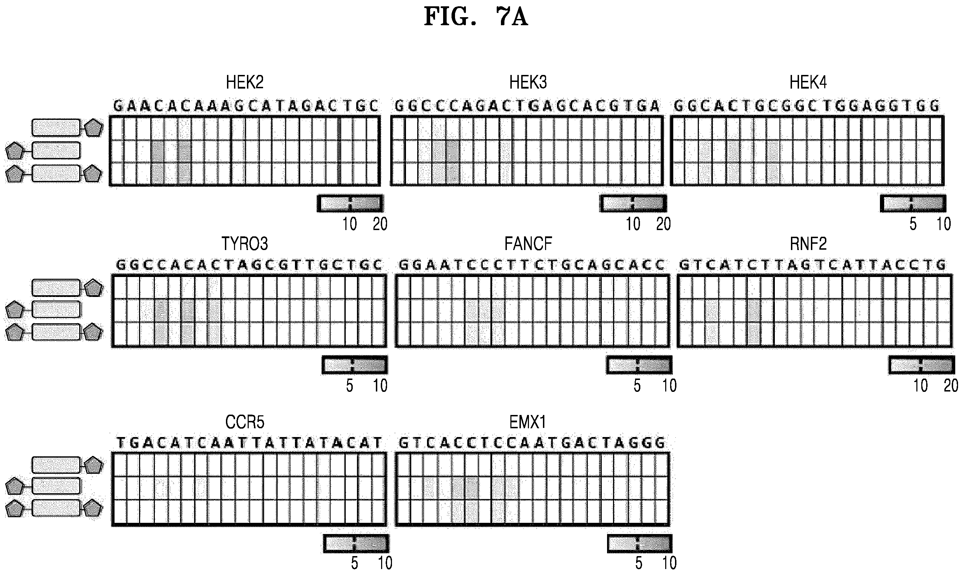

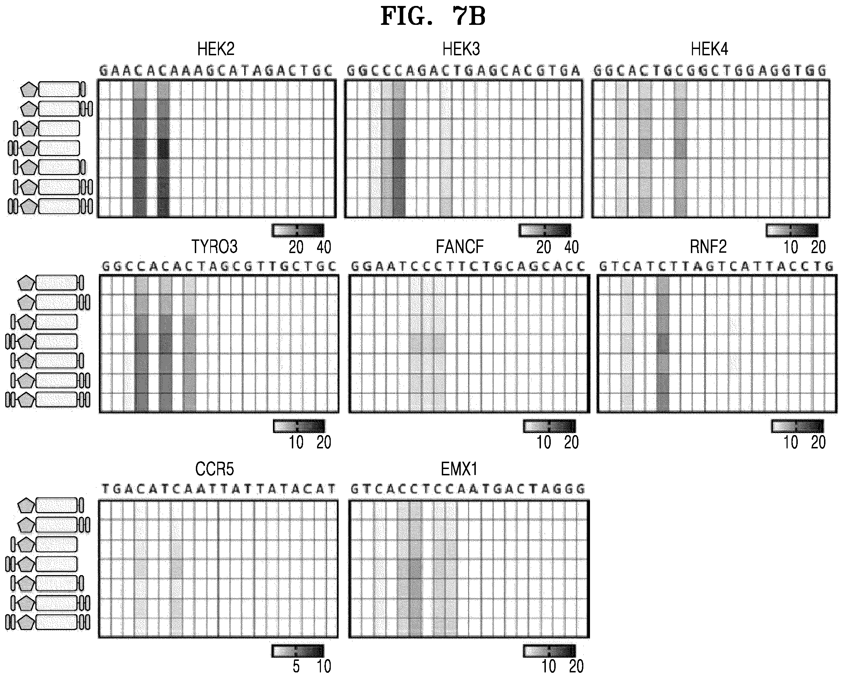

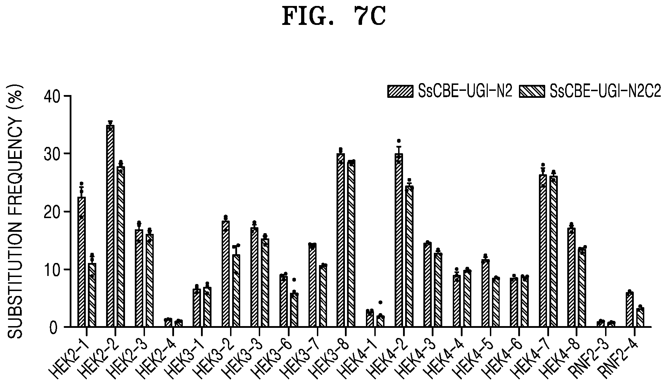

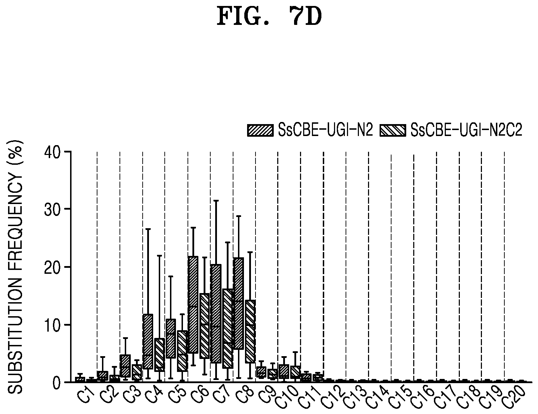

   FIG. 7A is a graph showing cytosine base editing efficiency of each CRISPR-Cas system including a Cas9(D10A)-SsdA fusion protein (SsdA-C), an SsdA-Cas9(D10A) fusion protein (SsdA-N, SsCBE), or an SsdA-Cas9(D10A)-SsdA(SsdA-NC) fusion protein, at HEK2, HEK3, HEK4, RNF2, FANCF, TYRO3, CCR5, or EMX1; FIG. 7B is a graph showing cytosine base editing efficiency of each CRISPR-Cas system including a fusion protein having one or two UGIs bound to the C-terminus, the N-terminus, and both the C-terminus and the N-terminus of the SsdA-Cas9(D10A) fusion protein (SsdA-N, SsCBE), at HEK2, HEK3, HEK4, RNF2, FANCF, TYRO3, CCR5, or EMX1; FIG. 7C is a graph showing cytosine base editing efficiency at 20 target sites (HEK2-1, HEK2-2, HEK2-3, HEK2-4, HEK3-1, HEK3-2, HEK3-3, HEK3-6, HEK3-7, HEK3-8, HEK4-1, HEK4-2, HEK4-3, HEK4-4, HEK4-5, HEK4-6, HEK4-7, HEK4-8, RNF2-3, and RNF2-4) for comparison of base editing efficiency with respect to: a UGI-UGI-SsdA-Cas9(D10A) fusion protein(SsCBE-UGI-N2) in which two UGIs with significantly high base editing efficiency are bound to the N-terminus; and a UGI-UGI-SsdA-Cas9(D10A)-UGI-UGI fusion protein (SsCBE-UGI-N2C2) in which two UGIs are bound to both the N-terminus and the C-terminus; and FIG. 7D is a graph showing the results of analyzing editing window, which is a technical characteristic of the base editing efficiency, with respect to the base editing efficiency for the 20 target sites shown in FIG. 7C.
   FIG. 8 is a graph showing substitution frequency (%) of a UGI-UGI-SsdA-Cas9(D10A) fusion protein (SsCBE-UGI-N2), BE3, and BE4max at a target site (HEK2, HEK3, HEK2-2, HEK3-8, HEK4-2, or HEK4-7 site).
   FIG. 9 is a graph showing base editing efficiency and indel formation efficiency of each CRISPR-Cas system including a Cas9(D10A)-SsdA(G54D)-UGI fusion protein, a Cas9(D10A)-SsdA(G54D) fusion protein, a dCas9-SsdA(G54D)-UGI fusion protein, or a dCas9-SsdA(G54D) fusion protein, at HEK2, HEK3, and HEK4.

### Mode for the Invention

Hereinafter, the present disclosure will be described in more detail with reference to Examples below. However, these Examples are for illustrative purposes only, and the scope of the present disclosure is not intended to be limited by these Examples.

### Example 1. Plasmid cloning

A gBlock double-stranded DNA fragment (Integrated DNA Technologies) encoding His6-SsdA having an amino acid sequence of SEQ ID NO: 1 and SsdAI having an amino acid sequence of SEQ ID NO: 3 and the pET-28b(+) DNA bacterial expression vector (Novagen) were treated with Xbal and Xhol restriction enzymes (New England Biolabs), respectively, at 37 °C for 3 hours. The linearized pET-28b vector was then purified using agarose gel extraction (Geneall) and ligated to the gBlock double-stranded DNA fragment using the Quick Ligase Kit (New England Biolabs).

Next, using the pCMV plasmid DNA, the coding sequences of Cas9 and UGI were obtained through PCR amplification, and the SsdA sequence was amplified from the gBlock by using Gibson Assembly Master Mix (New England Biolabs), and then sub-cloned into the pCMV plasmid.

The amino acid sequence of the PAAR-domain-containing protein of SEQ ID NO: 1 and the amino acid sequence of the SsdAI of SEQ ID NO: 3 are shown in Table 2.

**[Table 2]**

| | Amino acid sequence | SEQ ID NO: |
|---|---|---|
| SsdA protein (PAAR-domain-containi ng protein) | MSAAARVNDPIEHTGSLTGLLAGLAIGAIGAALVVGTGGLAA VAIVGASAATGAGVGQLIGSLSCCNHQTGQIVSGSSNVYIN GEPAARAHADQAKCDEHTSRPQVIAQGSSNVYINGHPAAR VGDRTACDAKIVVGSSNVFIGGGTETTDPINPEVPELLERSI LLVGLASAVVLASPVIVIAGLVGGIAGGTVGSMGGAQLFGE GTDGQKLMAFGGALLGGGLGAKGGKWFDTRYDIKVQGVG SNLGNLKITPKGAAKVSNIAESEAALGRASQARADLPQSKE LKVKTVSSNDKKTLSGWGNKKPEGYERISAEQVKAKSEEIG HEVKSHPYDRDYKGQYFSSHAEKQMSIASPNHPLGVSKPM CTDCQGYFSQLAKYSKVEQTVADPKAIRIFKTDGSVETIMR SE | SEQ ID NO: 1 |
| SsdAl protein | MNNKSKVLIEKLLLEVAKSPEGELILPLRKLLWNTITEDETAA KKKAILTALDVMCVRQGVNFWIKKFGDNEPLNYILNIALETA EGKFDESKALGLRDEFYVSIVEDQEYEVEEYPAMFVGHAA ANTIARAVDDFQFEPYDHRVDRDLDPEGFESSYLVASAFAG GLSEDGDPKLRRAFWEWYLSIAVPQVV | SEQ ID NO: 3 |

### Example 2. Purification of SsdA

The SsdA protein cloned in Example 1 was purified using *E*. *coli* BL21.

More specifically, the pET-28b-His6-SsdA-SsdAl was introduced into the *E. coli* BL21 by using 0.5 mM IPTG, and a complex protein of His6-SsdA and SsdAI was purified using Ni-NTA agarose beads (Qiagen). Next, to separate the His6-SsdA from the SsdAl, the complex protein of His6-SsdA and SsdAI was denatured with denaturing buffer (8 M urea, 50 mM Tris-HCl pH 7.5, 500 mM NaCl, and 1 mM DTT) and then cultured at 4 °C for 16 hours. The suspension buffer containing the denatured complex protein was mixed with Ni-NTA agarose beads (Qiagen) and loaded onto a gravity-flow column to remove unbound SsdAl. Afterwards, denaturing buffer with decreasing concentrations of elements (6 M, 4 M, 2 M, 1 M, and 0 M) were treated for refolding of the SsdA. The refolded proteins bound to the Ni-NTA agarose beads were eluted with elution buffer containing 300 mM imidazole, and the eluted proteins were dialyzed using 20 mM Tris-HCl pH 7.5, 200 mM NaCl, 1 mM DTT, and 40 % (w/v) glycerol, and concentrated using the Amicon Ultra-15 Centrifugal Filter Unit (Millipore). Then, the concentration of the His6-SsdA protein was analyzed by SDS-PAGE.

### Example 3. Cell culture and transfection

HEK293T cells (ATCC CRL-11268) were maintained in a Dulbecco's modified Eagle's medium (DMEM) supplemented with 10 % fetal bovine serum (FBS) and 1% penicillin/streptomycin (Welgene,) and the HEK293T cells were seeded in a TC-treated 48-well plate (Corning Life Sciences) at a density of 6 × 10⁴ cells per well. Twenty-four hours after seeding, transfection was performed at about 60 % cell confluency by using 500 ng of plasmids (250 ng of Cas9-SsdA expression plasmid and 250 ng of gRNA expression plasmid) and 1.5 uL of Lipofectamine 2000 (Thermo Fisher Scientific). Then, the transfected cells were incubated at 37 °C for 3 days, and the genomic DNA was prepared by directly lysing the cells with lysis buffer (10 mM Tris-HCl at pH 7.5, 0.05 % SDS, 100 mg/mL proteinase K; QIAGEN). The cell lysate was incubated at 56 °C for 30 minutes, and then additionally incubated at 99 °C for 15 minutes to inactivate Proteinase K.

### Example 4. Targeted deep sequencing and data analysis

A target region was amplified by PCR (two or three times in total) and sequenced using the Illumina MiniSeq or iSeq 100 sequencing system.

More specifically, 3 mL of cell lysate or 1 mL of isolated genomic DNA was subjected to a primary PCR, and then 1 mL of the primary PCR product was used for a second PCR. Illumina TruSeq HT dual index adapter sequences were attached to a pair of index PCR primers by using 1 mL of the secondary PCR product. The size of PCR amplicons was confirmed on a 2 % agarose gel, and the amplicons were sequenced using the Illumina MiniSeq or iSeq 100 sequencing system. Targeted deep sequencing analysis was performed using MAUND (https://github.com/ibscge/maund), and all results were confirmed with Cas-Analyzer (http://www.rgenome.net/cas-analyzer/).

### Example 5. Confirmation of deamination of cytosine in single-stranded DNA of SsdA

To determine whether cytosine bases in the single-stranded DNA of SsdA was converted to uracil, single-stranded DNA containing FAM was treated with SsdA.

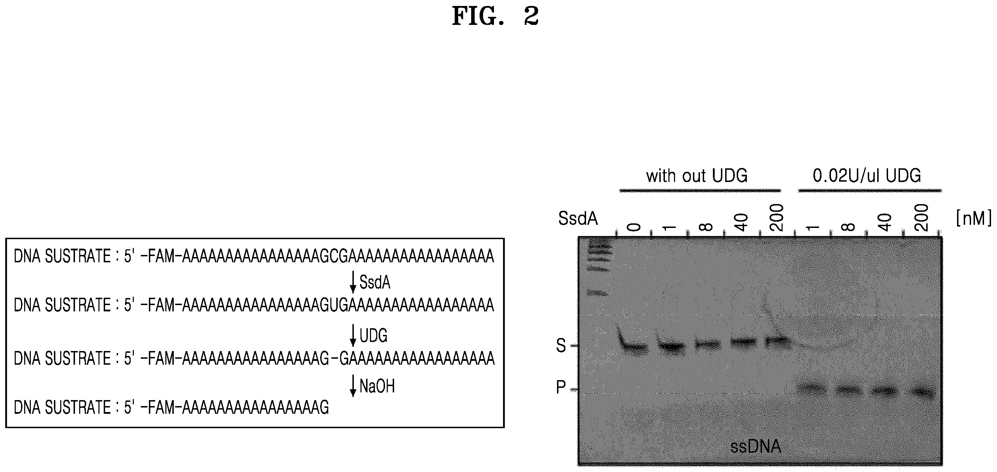

More specifically, single-stranded DNA having the base sequence of SEQ ID NO: 4 (5'- Aaaaaaaaaaaaaaagcgaaaaaaaaaaaaaaaaa-3) was treated with 1 to 200 nM of SsdA at 37 °C for 1 hour, and then uracil DNA glycosylase (UDG) was treated therewith at 37 °C for 30 minutes to remove the DNA bases that have been changed to uracil, thereby creating abasic sites. Afterwards, 100 mM NaOH was treated therewith, followed by incubation at 95 °C for 2 minutes to cleave the abasic sites. Whether or not the cleavage was successful was confirmed through western blot, and the results are shown in FIG. 2.

FIG. 2 is a diagram describing a process of deaminating single-stranded DNA having the base sequence of SEQ ID NO: 4 by treatment with SsdA and cleaving the single-stranded DNA by treatment with UDG and NaOH, and shows an image showing the results of western blot to confirm deamination of the SsdA.

As shown in FIG. 2, it was confirmed that DNA cleavage occurred only when the single-stranded DNA was treated with SsdA, UDG, and NaOH. This suggests that the SsdA was able to effectively deaminate cytosine in the single-stranded DNA and convert it to uracil.

### Example 6. Determination of base editing efficiency of CRISPR-Cas system including Cas protein, SsdA, and guide polynucleotide

To determine the base editing efficiency of the CRISPR-Cas system including Cas proteins, SsdA, and guide polynucleotides, the CRISPR-Cas system including SsdA, dCas9, and gRNA was treated with target DNA (RNF2 and HEK2) to determine whether deamination of cytosine was formed. The base sequences of the target DNAs (RNF2 and HEK2) are shown in Table 3.

**[Table 3]**

| Target DNA | Target site | SEQ ID NO: |
|---|---|---|
| RNF2 | GTC3ATC6TTAGTC12ATTACCTGAGG | SEQ ID NO: 5 |
| HEK2 | GAAC4AC6AAAGC11ATAGACTGCGGG | SEQ ID NO: 6 |

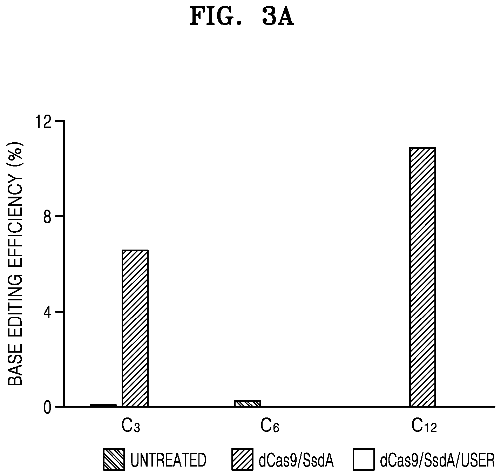

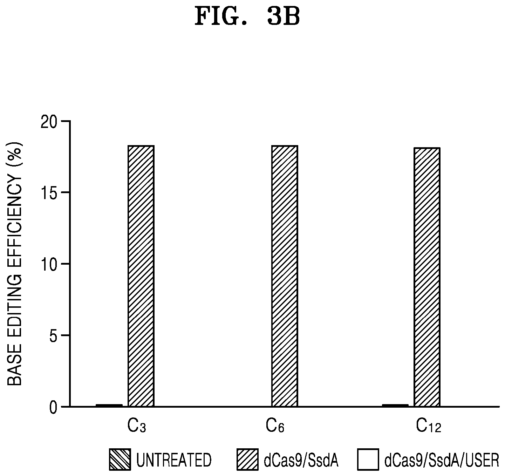

More specifically, RNF2 DNA and HEK2 DNA were each treated with 100 nM Cas9, 300 nM sgRNA, and 40 nM SsdA, and then incubated at 37 °C for 8 hours to induce conversion of cytosine to uracil at the target site of the target DNA. Then, after 8 hours, the sgRNA, Cas9, and SsdA were removed by treatment with RNase and Protease K. Then, DNA purification was performed using the Qiagen DNA extraction kit. Afterwards, PCR was performed using primers including the target site, and the base editing efficiency was measured through deep sequencing, and the results are shown in FIG. 3.

FIG. 3 is a graph showing the base editing efficiency of the CRISPR-Cas system at the target site of the target DNA, the CRISPR-Cas system including a Cas protein, SsdA, and a guide polynucleotide.

FIG. 3A is a graph showing the base editing efficiency of a CRISPR-Cas system, which includes a Cas protein, SsdA, and guide polynucleotide, at a target site of RNF2 DNA, and FIG. 3B is a graph showing the base editing efficiency of a CRISPR-Cas system, which includes a Cas protein, SsdA, and a guide polynucleotide, at a target site of HEK2 DNA.

As shown in FIG. 3A, when gRNA targeting RNF2 was used, the conversion efficiency of cytosine to uracil was confirmed to be about 7 % at the C₃ position and about 11 % at the C₁₂ position. In addition, as shown in FIG. 3B, when gRNA targeting HEK2 was used, the conversion efficiency of cytosine to uracil was confirmed to be about 18 to 19 % at the C₄, C₆, and C₁₁ positions.

Considering that the CRISPR-Cas system can cause gene editing only at one strand of the DNA double strands and thus has a maximum base editing efficiency of 50 %, the results above indicate a considerably high level of base editing efficiency.

Furthermore, to confirm whether the base conversion caused by the CRISPR-Cas system including the Cas protein, SsdA, and guide polynucleotide is a conversion from cytosine to uracil, the deaminated DNA was additionally treated with uracil-specific excision reagent (USER), which is a uracil-specific excision enzyme, and the target site was amplified through PCR. As a result, it was confirmed that the number of reads where cytosine was converted to uracil was eliminated.

These results suggest that the CRISPR-Cas system effectively converts cytosine to uracil.

### Example 7. Confirmation of cytotoxicity of fusion proteins including Cas protein and SsdA

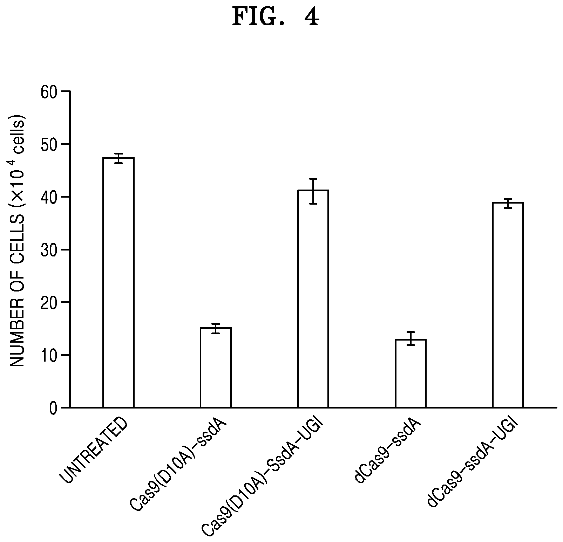

The cytotoxicity of the fusion protein including Cas protein, SsdA, and UGI was confirmed, and the results are shown in FIG. 4.

To determine whether SsdA included in the CRISPR-Cas system is toxic in eukaryotic cells, a plasmid expressing a protein combined with Cas9 (D10A) was manufactured. Then, HEK293 cells were seeded in a 48-well plate at a density of 6 x 10⁴ cells/well, and the plasmid was transfected into the HEK293 cells. 48 hours after transfection, live cells were trypsinized and the number of HEK293 cells was counted using a hemocytometer.

FIG. 4 is a graph showing the cytotoxicity of each of a Cas9(D10A)-SsdA fusion protein, a Cas9(D10A)-SsdA-UGI fusion protein, a dCas9-SsdA fusion protein, and a dCas9-SsdA-UGI fusion protein.

As shown in FIG. 4, it was confirmed that the fusion proteins including Cas9, SsdA, and UGI had almost no cytotoxicity.

### Example 8. Confirmation of base editing efficiency and indel formation efficiency of CRISPR-Cas system including fusion protein including Cas protein and SsdA

To determine whether SsdA included in the CRISPR-Cas system can effectively induce cytosine deamination in eukaryotic cells, a plasmid expressing a protein bound to Cas9 was manufactured and transfected into HEK293 cells. Then, the base sequences of target sites (e.g., HEK2, HEK3, HEK4, RNF2, EPAS1_e2, EPAS1_e5, HIF_e8, HIF_e9, or TFPi site) were amplified by PCR from the transfected cells, and then analyzed for the introduction of mutations and indel formation using next-generation sequencing (NGS), and the results are shown in FIG. 5.

For use as the Cas9, spCas9 (Cas9 (D10A)) or cjCas9 (D8A) was used. Uracil-DNA glycosylase (UDG) knockdown cell line (UDG KD) was prepared by transfecting HEK293 cells with a mixture prepared by mixing an shRNA-expressing plasmid (5'-GTCTACAGACATAGAGGATTT -3: SEQ ID NO: 7), which knocks down UDG, and an HIV-based packaging plasmid (including the following genes: Gag/Pol, Rev, and VSV-G) with Lipofectamine 3000 (Invitrogen) reagent in Opti-MEM (Invitrogen).

FIG. 5 is a graph showing the base editing efficiency and indel formation efficiency of the CRISPR-Cas system including the Cas9-SsdA fusion protein:

FIG. 5A is a graph showing the base editing efficiency and indel formation efficiency of each CRISPR-Cas system including a Cas9(D10A)-SsdA fusion protein, a Cas9(D10A)-SsdA-UGI fusion protein, a dCas9-SsdA fusion protein, or a dCas9-SsdA-UGI fusion protein, at HEK2, HEK3, HEK4, and RNF2; FIG. 5B is a graph showing the base editing efficiency and indel formation efficiency of each CRISPR-Cas system including a cjCas9(D8A)-SsdA fusion protein, a cjCas9(D8A)-SsdA-UGI fusion protein, or a cjCas9(L58Y/D900K)(D8A)-SsdA-UGI fusion protein, at EPAS1_e2, EPAS1_e5, HIF_e8, HIF_e9, and TFPi; and FIG. 5C is a graph showing the base editing efficiency and indel formation efficiency of each CRISPR-Cas system including a cjCas9(D8A)-SsdA fusion protein, a cjCas9(D8A)-SsdA-UGI fusion protein, or a cjCas9(L58Y/D900K)(D8A)-SsdA-UGI fusion protein, at EPAS1_e2, EPAS1_e5, HIF_e8, HIF_e9, and TFPi in the UDG expression-KD cell line.

As shown in FIG. 5A, it was confirmed that the Cas9(D10A)-SsdA-UGI caused cytosine base editing with an efficiency of 4 to 11 % at all of the HEK2, HEK3, HEK4, and RNF2 sites, and that indels of the base sequences were formed at the target sites. In addition, it was confirmed that the dCas9-SsdA-UGI caused cytosine base editing at base correction occurred with an efficiency of 1 to 3.5 %, and that indels were not formed.

As shown in FIG. 5B, it was confirmed that both cjCas9(D8A)-SsdA-UGI and cjCas9(L58Y/D900K)(D8A)-SsdA-UGI caused base editing at an efficiency of about 3 %, demonstrating improved base editing efficiency compared to the fusion protein without UGI binding.

As shown in FIG. 5C, in the UDG KD cell line, both cjCas9(D8A)-SsdA-UGI and cjCas9(L58Y/D900K)(D8A)-SsdA-UGI showed a base editing efficiency of about 15 %, confirming a significantly improved base editing efficiency compared to cjCas9(D8A)-SsdA fusion protein without UGI binding.

Table 4 shows the results of analyzing the base-edited sequences generated by Cas9(D10A)-SsdA-UGI and HEK2-targeting gRNA.

**[Table 4]**

| Sequence | Read | SEQ ID NO: |
|---|---|---|
| GGAAACTGGAACACAAAGCATAGACTGCGGG GCGGGCCA | 3,912 (WT) | SEQ ID NO: 8 |
| GGAAACTGGAACATAAAGCATAGACTGCGGG GCGGGCCA | 237 | SEQ ID NO: 9 |
| GGAAACTGGAATATAAAGCATAGACTGCGGG GCGGGCCA | 103 | SEQ ID NO: 10 |
| GGAAACTGGAATACAAAGCATAGACTGCGGG GCGGGCCA | 61 | SEQ ID NO: 11 |
| GGAAACTGGAACACAAAGTATAGACTGCGGG GCGGGCCA | 29 | SEQ ID NO: 12 |

### Example 9. Confirmation of base editing efficiency according to binding location of SsdA and Cas protein

Uracil-DNA glycosylase (UDG) is a well-known protein that repairs cytosine deamination in intracellular DNA. To confirm precisely that SsdA actually causes cytosine deamination in intracellular DNA, a HEK293 cell line with UDG knock-out (HEK293 UDG-KO) was prepared.

To determine the base editing efficiency and indel formation efficiency of fusion proteins in which SsdA is bound to the C-terminus, the N-terminus, and both the N-terminus and the C-terminus of a Cas protein, fusion proteins were prepared by varying the position of SsdA, and the base editing efficiency and indel formation efficiency were confirmed therefrom.

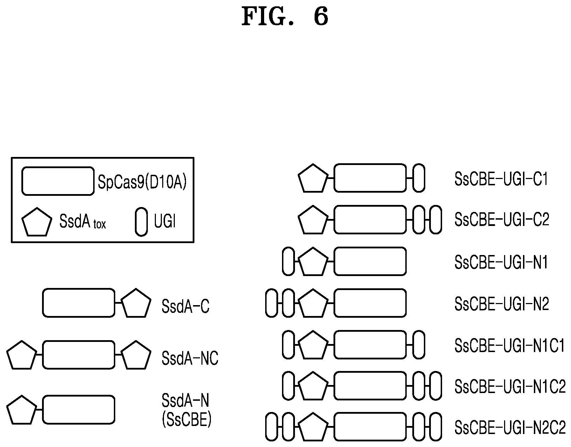

More specifically, a pCMV plasmid as shown in FIG. 6 was manufactured and introduced into HEK293 UDG-KO cells together with gRNA for each target site according to Example 3. Then, the base sequences of target sites (e.g., HEK2, HEK3, HEK4, RNF2, FANCF, TYRO3, CCR5, or EMX1 site) were amplified by PCR from the transfected cells, and then analyzed for the introduction of mutations and indel formation using next-generation sequencing (NGS), and the results are shown in FIG. 7.

FIG. 6 is a schematic diagram of a plasmid for producing a fusion protein in which SsdA and uracil glycosylase inhibitor (UGI) are bound to the C-terminus, N-terminus, and both the N-terminus and the C-terminus of the Cas protein.

FIG. 7 is a graph confirming the base editing efficiency according to the binding location of SsdA and Cas protein.

FIG. 7A is a graph showing the cytosine base editing efficiency of each CRISPR-Cas system including Cas9(D10A)-SsdA fusion protein (SsdA-C), SsdA-Cas9(D10A) fusion protein (SsdA-N, SsCBE), or SsdA-Cas9(D10A)-SsdA(SsdA-NC) fusion protein, at HEK2, HEK3, HEK4, RNF2, FANCF, TYRO3, CCR5, or EMX1; FIG. 7B is a graph showing the cytosine base editing efficiency of each CRISPR-Cas system including a fusion protein having one or two UGIs bound to the C-terminus, the N-terminus, and both the C-terminus and the N-terminus of the SsdA-Cas9(D10A) fusion protein (SsdA-N, SsCBE), at HEK2, HEK3, HEK4, RNF2, FANCF, TYRO3, CCR5, or EMX1; FIG. 7C is a graph showing the cytosine base editing efficiency at 20 target sites (HEK2-1, HEK2-2, HEK2-3, HEK2-4, HEK3-1, HEK3-2, HEK3-3, HEK3-6, HEK3-7, HEK3-8, HEK4-1, HEK4-2, HEK4-3, HEK4-4, HEK4-5, HEK4-6, HEK4-7, HEK4-8, RNF2-3, and RNF2-4) for comparison of base editing efficiency with respect to: UGI-UGI-SsdA-Cas9(D10A) fusion protein(SsCBE-UGI-N2)in which two UGIs with significantly high base editing efficiency are bound to the N-terminus; and UGI-UGI-SsdA-Cas9(D10A)-UGI-UGI fusion protein (SsCBE-UGI-N2C2) in which two UGIs are bound to both the N-terminus and the C-terminus; and FIG. 7D is a graph showing the results of analyzing editing window, which is a technical characteristic of the base editing efficiency, with respect to the base editing efficiency for the 20 target sites shown in FIG. 7C.

The base sequences of the target sites of the target genes are shown in Table 5.

**[Table 5]**

| Target DNA | Target site | SEQ ID NO: |
|---|---|---|
| HEK2 | GAACACAAAGCATAGACTGC | 18 |
| HEK2-1 | CCAGCCCGCTGGCCCTGTAA | 19 |
| HEK2-2 | GCTGGCCCTGTAAAGGAAAC | 20 |
| HEK2-3 | GTTTCCTTTACAGGGCCAGC | 21 |
| HEK2-4 | GCACTTGTTTGCAGCTATTC | 22 |
| HEK3 | GGCCCAGACTGAGCACGTGA | 23 |
| HEK3-1 | CTGCTTCTCCAGCCCTGGCC | 24 |
| HEK3-2 | CCCTGGCCTGGGTCAATCCT | 25 |
| HEK3-3 | GACTGAGCACGTGATGGCAG | 26 |
| HEK3-6 | CTTCCTCCAGAGGGCGTCGC | 27 |
| HEK3-7 | CAGGACAGCTTTTCCTAGAC | 28 |
| HEK3-8 | CAGCTCCTGCACCGGGATAC | 29 |
| HEK4 | GGCACTGCGGCTGGAGGTGG | 30 |
| HEK4-1 | GGGGCACCGCGGCGCCCCGG | 31 |
| HEK4-2 | GCGGCGCCCCGGTGGCACTG | 32 |
| HEK4-3 | CGCCCCGGTGGCACTGCGGC | 33 |
| HEK4-4 | TCCCTTCCTTCCACCCAGCC | 34 |
| HEK4-5 | CCCTGCCTGTCATCCTGCTT | 35 |
| HEK4-6 | GCAGTGCCACCGGGGCGCCG | 36 |
| HEK4-7 | CTCCAGCCGCAGTGCCACCG | 37 |
| HEK4-8 | ACCTCCAGCCGCAGTGCCAC | 38 |
| RNF2 | GTCATCTTAGTCATTACCTG | 39 |
| RNF2-3 | TACACGTCTCATATGCCCCT | 40 |
| RNF2-4 | TCAACCATTAAGCAAAACAT | 41 |
| EMX1 | GTCACCTCCAATGACTAGGG | 42 |
| FANCF | GGAATCCCTTCTGCAGCACC | 43 |
| TYRO3 | GGCCACACTAGCGTTGCTGC | 44 |
| CCR5 | TGACATCAATTATTATACAT | 45 |

As shown in FIG. 7A, SsdA-Cas9(D10A)(SsdA-C) in which SsdA is bound to the C-terminus of Cas had slightly reduced cytosine base editing efficiency compared to the cases where SsdA is bound to the N-terminus or both the N-terminus and the C-terminus. On the other hand, when SsdA is bound to the N-terminus of Cas (SsdA-N, SsCBE), it was confirmed that the base editing efficiency increased.

As shown in FIG. 7B, it was confirmed that additional binding of UGI to SsdA-Cas9(D10A)(SsdA-N, SsCBE), which has high base editing efficiency, increased the base editing efficiency compared to the absence of UGI, and that the base editing efficiency was significantly higher for UGI-UGI-SsdA-Cas9(D10A) fusion protein (SsCBE-UGI-N2) with two UGIs at the N-terminus or for UGI-UGI-SsdA-Cas9(D10A)-UGI-UGI fusion protein (SsCBE-UGI-N2C2) with two UGIs at the N-terminus and two UGIS at the C-terminus.

As shown in FIG. 7C, as a result of comparing at the 20 target sites for either UGI-UGI-SsdA-Cas9(D10A) fusion protein (SsCBE-UGI-N2) having the highest base editing efficiency with two UGIs at the N-terminus or UGI-UGI-SsdA-Cas9(D10A)-UGI-UGI fusion protein (SsCBE-UGI-N2C2) with two UGIs at the N-terminus and two UGIs at the C-terminus, it was confirmed that the UGI-UGI-SsdA-Cas9(D10A) fusion protein (SsCBE-UGI-N2) with two UGIs at the N-terminus showed slightly higher base editing efficiency.

As shown in FIG. 7D, as a result of comparing the editing window at the 20 target sites for either UGI-UGI-SsdA-Cas9(D10A) fusion protein (SsCBE-UGI-N2) having the highest base editing efficiency with two UGIs at the N-terminus or UGI-UGI-SsdA-Cas9(D10A)-UGI-UGI fusion protein (SsCBE-UGI-N2C2) with two UGIs at the N-terminus and two UGIs at the C-terminus, it was confirmed that the highest base editing efficiency was achieved at positions between 4 bp and 8 bp from the 5'-end of the gRNA target base sequence.

### Example 10. Confirmation of base editing ability by using UGI-UGI-SsdA-Cas9(D10A) fusion protein (SsCBE-UGI-N2) in wild-type HEK293 cells

It was confirmed whether the base editing efficiency was observed in wild-type HEK293 cells according to the UGI-UGI-SsdA-Cas9(D10A) fusion protein (SsCBE-UGI-N2), which was confirmed to have the highest base editing ability.

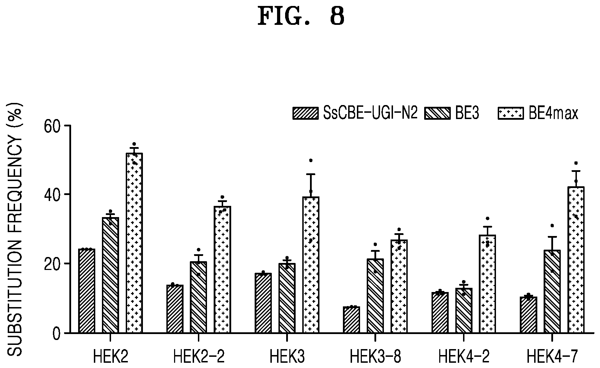

More specifically, the UGI-UGI-SsdA-Cas9(D10A) fusion protein (SsCBE-UGI-N2) and the existing BE3 or BE4max were introduced into HEK293 cells together with gRNA for each target site according to Example 3. Then, the base sequences of target sites (e.g., HEK2, HEK3, HEK2-2, HEK3-8, HEK4-2, or HEK4-7 site) were amplified by PCR from the transfected cells, and then analyzed for the introduction of mutations and indel formation using next-generation sequencing (NGS), and the results are shown in FIG. 8.

FIG. 8 is a graph showing the substitution frequency (%) of the UGI-UGI-SsdA-Cas9(D10A) fusion protein (SsCBE-UGI-N2), BE3, and BE4max at the target site (HEK2, HEK3, HEK2-2, HEK3-8, HEK4-2, or HEK4-7 site).

As shown in FIG. 8, the UGI-UGI-SsdA-Cas9(D10A) fusion protein (SsCBE-UGI-N2) successfully exhibited the base editing efficacy at 6 target sites (e.g., HEK2, HEK3, HEK2-2, HEK3-8, HEK4-2, or HEK4-7 site).

### Example 11. Confirmation of cytotoxicity and base editing ability of CRISPR-Cas system using SsdA mutant

To determine the cytotoxicity and base editing ability of SsdA mutant, inactivated SsdA was prepared and its effects were confirmed.

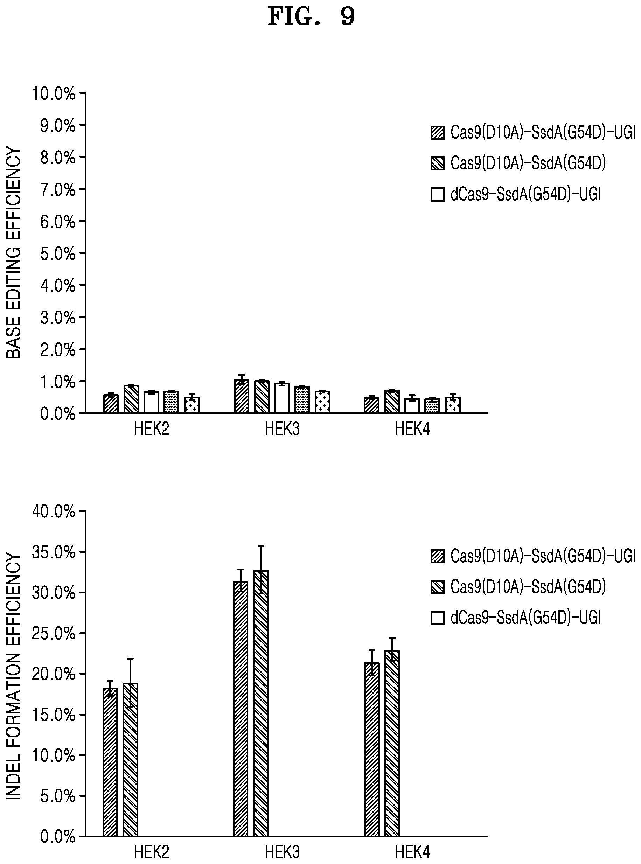

More specifically, after introducing a G54D mutation into the catalytically active site of SsdA, the mutated SsdA protein was bound to Cas9 and its intracellular function was confirmed. Then, the results are shown in FIG. 9. The G54D mutation at the catalytic active site of SsdA is a G302D mutation in the amino acid sequence of SEQ ID NO: 1 (PAAR domain-containing protein). Specifically, the G54D mutation at the catalytically active site of the SsdA may have the sequence of SEQ ID NO: 17. The amino acid sequence of the G54D mutation at the catalytically active site of the SsdA is shown in Table 6.

**[Table 6]**

| | Amino acid sequence | SEQ ID NO: |
|---|---|---|
| G54D mutation sequence at catalytic site of SsdA | KVSNIAESEAALGRASQARADLPQSKELKVKTVSSNDKK TLSDWGNKKPEGYERISAEQVKAKSEEIGHEVKSHPYDR DYKGQYFSSHAEKQMSIASPNHPLGVSKPMCTDCQGYF SQLAKYSKVEQTVADPKAIRIFKTDGSVETIMRSE | 17 |

FIG. 9 is a graph showing the base editing efficiency and indel formation efficiency of each CRISPR-Cas system including Cas9(D10A)-SsdA(G54D)-UGI fusion protein, Cas9(D10A)-SsdA(G54D) fusion protein, dCas9-SsdA(G54D)-UGI fusion protein, or dCas9-SsdA(G54D) fusion protein, at HEK2, HEK3, and HEK4.

As shown in FIG. 9, it was confirmed that, when the G54D mutation of SsdA binds to Cas9 (D10A), indels were produced with a very high efficiency of about 18 to 34 %, regardless of the presence or absence of UGI. This suggests that the SsdA(G54D) can induce indels at the target site with higher efficiency, regardless of UGI function.

It was also confirmed that, when the SsdA(G54D) was used, not only was the intracellular toxicity significantly reduced, but also the size of the deleted base sequences was significantly increased.

Table 7 shows the results of analyzing the base-edited sequences caused by SsdA(G54D)-Cas9(D10A).

As shown in Table 7, as a result of analyzing indel sequences caused by SsdA(G54D)-Cas9(D10A), it was confirmed that, unlike Cas9 that usually forms a 1 bp deletion, the size of the deleted base sequences was significantly increased in the case of the SsdA(G54D)-Cas9(D10A). These results imply that the CRISPR-Cas system including SsdA(G54D) can increase the gene knock-out efficiency compared to the system using Cas9 alone.

**[Table 7]**

| Sequence | Read | SEQ ID NO: |
|---|---|---|
| AATTTTCCAGCCCGCTGGCCCTGTAAAGGA AACTGGAACACAAAGCATAGACTGCGGGGCG GGCCAGCCTGAATA | 1,010 (WT) | SEQ ID NO: 13 |
| AATTTTCCAGCCTGAATA | 503 | SEQ ID NO: 14 |
| AATTTTCCAGCCCGCTGGCCCTGTAAAGGAA ACTGGAACACAAAGCGGGGCGGGCCAGCCT GAATA | 354 | SEQ ID NO: 15 |
| AATTTTCCAGCCCGCTGGCCCTGTAAAGGAA ACTGGAACACAAAGCATA | 349 | SEQ ID NO: 16 |

## Claims

1. A fusion protein comprising a CRISPR-associated (Cas) protein and a bacterial toxin, wherein the bacterial toxin is single-stranded DNA deaminase toxin A (SsdA).

2. The fusion protein of claim 1, wherein the Cas protein is a Cas9 protein or a Cas12 protein.

3. The fusion protein of claim 1, wherein the SsdA is cytidine deaminase.

4. The fusion protein of claim 1, wherein the SsdA is inactivated SsdA.

5. The fusion protein of claim 1, wherein the SsdA comprises an amino acid sequence of SEQ ID NO: 1.

6. The fusion protein of claim 1, wherein the SsdA comprises a sequence having at least 85 % sequence identity with an amino acid sequence of SEQ ID NO: 1.

7. The fusion protein of claim 4, wherein the inactivated SsdA has an amino acid mutation at a catalytic active site of the SsdA.

8. The fusion protein of claim 4, wherein the inactivated SsdA has G302D, E349A, or an amino acid mutation corresponding thereto, in an amino acid sequence of SEQ ID NO: 1.

9. The fusion protein of claim 4, wherein the inactivated SsdA comprises an amino acid sequence of SEQ ID NO: 17.

10. The fusion protein of claim 4, wherein the inactivated SsdA comprises a sequence having at least 85 % sequence identity with an amino acid sequence of SEQ ID NO: 17.

11. The fusion protein of claim 1, wherein the bacterial toxin is bound to the C-terminus, N-terminus, or both C-terminus and N-terminus of the Cas protein.

12. The fusion protein of claim 1, further comprising a DNA glycosylase inhibitor.

13. The fusion protein of claim 12, wherein the DNA glycosylase inhibitor comprises a thymine glycosylase inhibitor, an uracil glycosylase inhibitor, an oxoguanine glycosylase inhibitor, or an alkylguanine DNA glycosylase inhibitor.

14. The fusion protein of claim 1, wherein the fusion protein has an editing window ranging from 1 bp to 20 bp from a 5'-end of a target sequence.

15. A polynucleotide encoding the fusion protein according to any one of claims 1 to 14.

16. A vector comprising the polynucleotide of claim 15.

17. A CRISPR-associated (Cas) protein system comprising: a fusion protein comprising a Cas protein and a bacterial toxin or a polynucleotide encoding the fusion protein; and a guide polynucleotide, wherein the bacterial toxin is single-stranded DNA deaminase toxin A (SsdA).

18. The CRISPR-Cas system of claim 17, wherein the guide polynucleotide comprises CRISPR RNA (crRNA) and trans-activating RNA (tracrRNA), and the guide polynucleotide comprises dual guide RNA or single-chain guide RNA (sgRNA).

19. The CRISPR-Cas system of claim 17, wherein the system forms deletion, insertion, substitution, or insertion and deletion (indel) of at least one nucleotide of nucleotide sequences of a target nucleic acid molecule.

20. The CRISPR-Cas system of claim 19, wherein the system forms deletion of nucleotides of 1 bp to 60 bp, insertion of nucleotides of 1 bp to 60 bp, or indel of nucleotides of 1 bp to 60 bp, in the nucleotide sequence of a target nucleic acid molecule.

21. **The** CRISPR-Cas system of claim 17, wherein the system has an editing window ranging from 1 bp to 20 bp from a 5'-end of a target sequence.

22. A method of editing a nucleic acid, the method comprising contacting a nucleic acid molecule with a CRISPR-Cas system, wherein the editing is to form deletion, insertion, substitution, or insertion and deletion (indel) of at least one nucleotide sequence of nucleotide sequences of a nucleic acid molecule, and the CRISPR-Cas system comprises: a fusion protein comprising a CRISPR-associated (Cas) protein and a bacterial toxin or a polynucleotide encoding the fusion protein; and a guide polynucleotide, wherein the bacterial toxin is single-stranded **DNA** deaminase toxin A (SsdA).

23. **The** method of claim 22, wherein the editing is deletion of nucleotides of 1 bp to 60 bp, insertion of nucleotides of 1 bp to 60 bp, or indel of nucleotides of 1 bp to 60 bp, in the nucleotide sequence of a nucleic acid molecule.
